# EUROPÄISCHE PATENTSCHRIFT

(11) **EP 2 465 869 B1**
(45) Veröffentlichungstag und Bekanntmachung des Hinweises auf die Patenterteilung: **23.10.2013**
(21) Anmeldenummer: 11182716.8
(22) Anmeldetag: 24.10.2008
(51) Int. Cl.: C07K 14/24, C12P 13/08, C07K 14/245, C07K 14/255, C12P 13/04, C12P 13/06, C12P 13/10, C12P 13/12, C12P 13/14, C12P 13/20, C12P 13/22, C12P 13/24, C12R 1/19

(54) **Verfahren zur Herstellung von L-Aminosäuren unter Verwendung von verbesserten Stämmen der Familie Enterobacteriaceae**
Method for manufacturing L-amino acids using improved strains of the enterobacteriaceae family
Procédé de fabrication d'aminoacides L en utilisant des troncs améliorés de la famille des enterobacteriaceae

(30) Priorität: 02.11.2007 DE 102007052270
(43) Veröffentlichungstag der Anmeldung: 20.06.2012
(62) Teilanmeldung aus: 08167494.7
(73) Patentinhaber: Evonik Degussa GmbH, 45128 Essen (DE)
(72) Erfinder: Rieping, Mechthild, 33619 Bielefeld (DE); Hermann, Thomas, 63776 Mömbris (DE)

(56) Entgegenhaltungen:
- EP-A- 1 548 120
- WO-A-03/008615
- WO-A-2004/033671
- KVINT KRISTIAN ET AL: "The bacterial universal stress protein: Function and regulation.", CURRENT OPINION IN MICROBIOLOGY, Bd. 6, Nr. 2, April 2003 (2003-04), Seiten 140-145, XP002505558, ISSN: 1369-5274
- NYSTROM THOMAS ET AL: "Effects of overproducing the universal stress protein UspA, in Escherichia coli K-12", JOURNAL OF BACTERIOLOGY, Bd. 178, Nr. 3, 1996, Seiten 927-930, XP002505557, ISSN: 0021-9193
- FAREWELL ANNE ET AL: "Role of the Escherichia coli FadR regulator in stasis survival and growth phase-dependent expression of the uspA, fad, and fab genes", JOURNAL OF BACTERIOLOGY, Bd. 178, Nr. 22, 1996, Seiten 6443-6450, XP002505562, ISSN: 0021-9193
- DIEZ A A ET AL: "A mutation in the ftsK gene of Escherichia coli affects cell-cell separation, stationary-phase survival, stress adaptation, and expression of the gene encoding the stress protein UspA", JOURNAL OF BACTERIOLOGY, Bd. 179, Nr. 18, 1997, Seiten 5878-5883, XP002505559, ISSN: 0021-9193
- PHADTARE SANGITA ET AL: "Role of CspC and CspE in regulation of expression of RpoS and UspA, the stress response proteins in Escherichia coli", JOURNAL OF BACTERIOLOGY, Bd. 183, Nr. 4, Februar 2001 (2001-02), Seiten 1205-1214, XP002505560, ISSN: 0021-9193
- DIEZ ALFREDO ET AL: "The universal stress protein A of Escherichia coli is required for resistance to DNA damaging agents and is regulated by a RecA/FtsK-dependent regulatory pathway", MOLECULAR MICROBIOLOGY, Bd. 36, Nr. 6, Juni 2000 (2000-06), Seiten 1494-1503, XP002505561, ISSN: 0950-382X
- PENG ET AL: "Effect of fadR gene knockout on the metabolism of Escherichia coli based on analyses of protein expressions, enzyme activities and intracellular metabolite concentrations", ENZYME AND MICROBIAL TECHNOLOGY, STONEHAM, MA, US, Bd. 38, Nr. 3-4, 1. Februar 2006 (2006-02-01), Seiten 512-520, XP005206442, ISSN: 0141-0229
- CHEN J ET AL: "Cloning and sequencing of the gene encoding universal stress protein from Escherichia coli O157:H7 isolated from Jack-in-a-Box outbreak", LETTERS IN APPLIED MICROBIOLOGY, Bd. 29, Nr. 2, August 1999 (1999-08), Seiten 103-107, XP002505563, ISSN: 0266-8254

### SEQUENCE LISTING

<110> Evonik Degussa GmbH
<120> Verfahren zur Herstellung von L-Aminosäuren unter Verwendung von verbesserten Stämmen der Familie Enterobacteriaceae
<130> 2007P00724 EP01
<160> 14
<170> PatentIn version 3.3
<210> 1
   <211> 435
   <212> DNA
   <213> Escherichia coli
<220>
   <221> CDS
   <222> (1) .. (435)
   <223> uspA-Kodierregion
<400> 1
   atg gct tat aaa cac att ctc atc gcg gtc gac ctc tcc ccg gaa agc      48
   Met Ala Tyr Lys His Ile Leu Ile Ala Val Asp Leu Ser Pro Glu Ser
   1               5                   10                  15
   aaa gtt ctg gta gag aaa gca gtc tct atg gct cgc ccc tac aat gcg      96
   Lys Val Leu Val Glu Lys Ala Val Ser Met Ala Arg Pro Tyr Asn Ala
               20                  25                  30
   aaa gtt tct ctg atc cac gta gat gta aac tac tct gac cta tac acc     144
   Lys Val Ser Leu Ile His Val Asp Val Asn Tyr Ser Asp Leu Tyr Thr
           35                  40                  45
   ggg ctt att gat gtg aat ctg ggt gat atg cag aaa cgc atc tct gaa     192
   Gly Leu Ile Asp Val Asn Leu Gly Asp Met Gln Lys Arg Ile Ser Glu
       50                  55                  60
   gag aca cat cat gca ctg acc gag ctt tcc act aat gca ggc tac cca     240
   Glu Thr His His Ala Leu Thr Glu Leu Ser Thr Asn Ala Gly Tyr Pro
   65                  70                  75                  80
   atc act gaa acc ctg agc ggc agc ggc gac ctg ggc cag gtt ctg gtc     288
   Ile Thr Glu Thr Leu Ser Gly Ser Gly Asp Leu Gly Gln Val Leu Val
                   85                  90                  95
   gat gca atc aag aaa tac gat atg gat ttg gtg gtt tgt ggt cac cac     336
   Asp Ala Ile Lys Lys Tyr Asp Met Asp Leu Val Val Cys Gly His His
               100                 105                 110
   cag gac ttc tgg agc aaa ctg atg tct tcc gca cgt cag ctg atc aac     384
   Gln Asp Phe Trp Ser Lys Leu Met Ser Ser Ala Arg Gln Leu Ile Asn
           115                 120                 125
   acc gtt cac gtt gat atg ctg att gtt ccg ctg cgc gac gaa gaa gaa     432
   Thr Val His Val Asp Met Leu Ile Val Pro Leu Arg Asp Glu Glu Glu
       130                 135                 140
   taa                                                                 435
<210> 2
   <211> 144
   <212> PRT
   <213> Escherichia coli
<400> 2
   Met Ala Tyr Lys His Ile Leu Ile Ala Val Asp Leu Ser Pro Glu Ser
   1               5                   10                  15
   Lys Val Leu Val Glu Lys Ala Val Ser Met Ala Arg Pro Tyr Asn Ala
               20                  25                  30
   Lys Val Ser Leu Ile His Val Asp Val Asn Tyr Ser Asp Leu Tyr Thr
           35                  40                  45
   Gly Leu Ile Asp Val Asn Leu Gly Asp Met Gln Lys Arg Ile Ser Glu
       50                  55                  60
   Glu Thr His His Ala Leu Thr Glu Leu Ser Thr Asn Ala Gly Tyr Pro
   65                  70                  75                  80
   Ile Thr Glu Thr Leu Ser Gly Ser Gly Asp Leu Gly Gln Val Leu Val
                   85                  90                  95
   Asp Ala Ile Lys Lys Tyr Asp Met Asp Leu Val Val Cys Gly His His
               100                 105                 110
   Gln Asp Phe Trp Ser Lys Leu Met Ser Ser Ala Arg Gln Leu Ile Asn
           115                 120                 125
   Thr Val His Val Asp Met Leu Ile Val Pro Leu Arg Asp Glu Glu Glu
       130                 135                 140
<210> 3
   <211> 435
   <212> DNA
   <213> Salmonella enterica
<220>
   <221> CDS
   <222> (1) .. (435)
   <223> uspA-Kodierregion
<400> 3
   atg gct tat aaa cac att ctt atc gca gtt gat ctc tcc ccg gaa agt       48
   Met Ala Tyr Lys His Ile Leu Ile Ala Val Asp Leu Ser Pro Glu Ser
   1               5                   10                  15
   aaa gtt ctg gtt gaa aaa gcg gtc tct atg gcg cgc ccc tac aac gcg       96
   Lys Val Leu Val Glu Lys Ala Val Ser Met Ala Arg Pro Tyr Asn Ala
               20                  25                  30
   aaa atc tcc ctc atc cac gtt gat gtg aac tat tca gac ctg tac acc      144
   Lys Ile Ser Leu Ile His Val Asp Val Asn Tyr Ser Asp Leu Tyr Thr
           35                  40                  45
   ggt ctg att gac gtc aat ctg ggc gat atg cag aaa cgt atc tcc aaa      192
   Gly Leu Ile Asp Val Asn Leu Gly Asp Met Gln Lys Arg Ile Ser Lys
       50                  55                  60
   gaa acc cac cac gcg ctg acc gag ctg tct acc aac gct ggc tac cct      240
   Glu Thr His His Ala Leu Thr Glu Leu Ser Thr Asn Ala Gly Tyr Pro
   65                  70                  75                  80
   atc act gaa acc ctg agc ggt agc ggc gat ttg ggc cag gtg ctg gtt      288
   Ile Thr Glu Thr Leu Ser Gly Ser Gly Asp Leu Gly Gln Val Leu Val
                   85                  90                  95
   gac gcc atc aag aaa tac gat atg gat ctg gtc gtt tgc ggt cat cac      336
   Asp Ala Ile Lys Lys Tyr Asp Met Asp Leu Val Val Cys Gly His His
               100                 105                 110
   cag gac ttc tgg agc aaa ctg atg tct tct gcg cgc cag ctg atc aac      384
   Gln Asp Phe Trp Ser Lys Leu Met Ser Ser Ala Arg Gln Leu Ile Asn
           115                 120                 125
   acc gtt cac gtt gac atg ctg att gtt ccg ctg cgc gac gaa gaa gag      432
   Thr Val His Val Asp Met Leu Ile Val Pro Leu Arg Asp Glu Glu Glu
       130                 135                 140
   taa                                                                  435
<210> 4
   <211> 144
   <212> PRT
   <213> Salmonella enterica
<400> 4
   Met Ala Tyr Lys His Ile Leu Ile Ala Val Asp Leu Ser Pro Glu Ser
   1 5 10 15
   Lys Val Leu Val Glu Lys Ala Val Ser Met Ala Arg Pro Tyr Asn Ala
   20 25 30
   Lys Ile Ser Leu Ile His Val Asp Val Asn Tyr Ser Asp Leu Tyr Thr
   35 40 45
   Gly Leu Ile Asp Val Asn Leu Gly Asp Met Gln Lys Arg Ile Ser Lys
   50 55 60
   Glu Thr His His Ala Leu Thr Glu Leu Ser Thr Asn Ala Gly Tyr Pro
   65 70 75 80
   Ile Thr Glu Thr Leu Ser Gly Ser Gly Asp Leu Gly Gln Val Leu Val
   85 90 95
   Asp Ala Ile Lys Lys Tyr Asp Met Asp Leu Val Val Cys Gly His His
   100 105 110
   Gln Asp Phe Trp Ser Lys Leu Met Ser Ser Ala Arg Gln Leu Ile Asn
   115 120 125
   Thr Val His Val Asp Met Leu Ile Val Pro Leu Arg Asp Glu Glu Glu
   130 135 140
<210> 5
   <211> 432
   <212> DNA
   <213> Photorhabdus luminescens
<220>
   <221> CDS
   <222> (1)..(432)
   <223> uspA-Kodierregion
<400> 5
   atg gct tac aaa cat att ctt gtt gcg gtt gat tta tcc ccg gaa agt      48
   Met Ala Tyr Lys His Ile Leu Val Ala Val Asp Leu Ser Pro Glu Ser
   1               5                   10                  15
   cag gtt ttg gtg aga aaa gct gtt tct atg gct aaa ccg gac aat gcc      96
   Gln Val Leu Val Arg Lys Ala Val Ser Met Ala Lys Pro Asp Asn Ala
               20                  25                  30
   aaa gtt tcc ctg atc cat gtt gat gtt aac tat tcc gat ctc tac acc     144
   Lys Val Ser Leu Ile His Val Asp Val Asn Tyr Ser Asp Leu Tyr Thr
           35                  40                  45
   ggt ttg att gat gtt aat ctc ggt gat atg cag caa cgt att tct gag     192
   Gly Leu Ile Asp Val Asn Leu Gly Asp Met Gln Gln Arg Ile Ser Glu
       50                  55                  60
   gag acc cgc agt gcg ctg aaa gct ctc tcg gca gat tca gcc tat gac     240
   Glu Thr Arg Ser Ala Leu Lys Ala Leu Ser Ala Asp Ser Ala Tyr Asp
   65                  70                  75                  80
   att cag gaa acc ctg agt ggt agc ggt gat ctg ggg caa gta ctc gtt     288
   Ile Gln Glu Thr Leu Ser Gly Ser Gly Asp Leu Gly Gln Val Leu Val
                   85                  90                  95
   gat gca att aag aaa tat ggc ata gat atg gtt gtt tgc ggt cat cat     336
   Asp Ala Ile Lys Lys Tyr Gly Ile Asp Met Val Val Cys Gly His His
               100                 105                 110
   cag gat ttc tgg agc aaa ttg atg tct tct gct cgt cag tta att aac     384
   Gln Asp Phe Trp Ser Lys Leu Met Ser Ser Ala Arg Gln Leu Ile Asn
           115                 120                 125
   acg gtg cat gtt gat atg ctg att gtt cct ctg cgt gat gat gaa tga     432
   Thr Val His Val Asp Met Leu Ile Val Pro Leu Arg Asp Asp Glu
       130                 135                 140
<210> 6
   <211> 143
   <212> PRT
   <213> Photorhabdus luminescens
<210> 7
   <211> 2463
   <212> DNA
   <213> Escherichia coli
<220>
   <221> CDS
   <222> (1)..(2463)
   <223> thrA-Kodierregion
<400> 7
   atg cga gtg ttg aag ttc ggc ggt aca tca gtg gca aat gca gaa cgt      48
   Met Arg Val Leu Lys Phe Gly Gly Thr Ser Val Ala Asn Ala Glu Arg
   1               5                   10                  15
   ttt ctg cgt gtt gcc gat att ctg gaa agc aat gcc agg cag ggg cag      96
   Phe Leu Arg Val Ala Asp Ile Leu Glu Ser Asn Ala Arg Gln Gly Gln
               20                  25                  30
   gtg gcc acc gtc ctc tct gcc ccc gcc aaa atc acc aac cac ctg gtg     144
   Val Ala Thr Val Leu Ser Ala Pro Ala Lys Ile Thr Asn His Leu Val
           35                  40                  45
   gcg atg att gaa aaa acc att agc ggc cag gat gct tta ccc aat atc     192
   Ala Met Ile Glu Lys Thr Ile Ser Gly Gln Asp Ala Leu Pro Asn Ile
       50                  55                  60
   agc gat gcc gaa cgt att ttt gcc gaa ctt ttg acg gga ctc gcc gcc     240
   Ser Asp Ala Glu Arg Ile Phe Ala Glu Leu Leu Thr Gly Leu Ala Ala
   65                  70                  75                  80
   gcc cag ccg ggg ttc ccg ctg gcg caa ttg aaa act ttc gtc gat cag     288
   Ala Gln Pro Gly Phe Pro Leu Ala Gln Leu Lys Thr Phe Val Asp Gln
                   85                  90                  95
   gaa ttt gcc caa ata aaa cat gtc ctg cat ggc att agt ttg ttg ggg     336
   Glu Phe Ala Gln Ile Lys His Val Leu His Gly Ile Ser Leu Leu Gly
               100                 105                 110
   cag tgc ccg gat agc atc aac gct gcg ctg att tgc cgt ggc gag aaa     384
   Gln Cys Pro Asp Ser Ile Asn Ala Ala Leu Ile Cys Arg Gly Glu Lys
           115                 120                 125
   atg tcg atc gcc att atg gcc ggc gta tta gaa gcg cgc ggt cac aac     432
   Met Ser Ile Ala Ile Met Ala Gly Val Leu Glu Ala Arg Gly His Asn
       130                 135                 140
   gtt act gtt atc gat ccg gtc gaa aaa ctg ctg gca gtg ggg cat tac     480
   Val Thr Val Ile Asp Pro Val Glu Lys Leu Leu Ala Val Gly His Tyr
   145                 150                 155                 160
   ctc gaa tct acc gtc gat att gct gag tcc acc cgc cgt att gcg gca     528
   Leu Glu Ser Thr Val Asp Ile Ala Glu Ser Thr Arg Arg Ile Ala Ala
                   165                 170                 175
   agc cgc att ccg gct gat cac atg gtg ctg atg gca ggt ttc acc gcc     576
   Ser Arg Ile Pro Ala Asp His Met Val Leu Met Ala Gly Phe Thr Ala
               180                 185                 190
   ggt aat gaa aaa ggc gaa ctg gtg gtg ctt gga cgc aac ggt tcc gac     624
   Gly Asn Glu Lys Gly Glu Leu Val Val Leu Gly Arg Asn Gly Ser Asp
           195                 200                 205
   tac tct gct gcg gtg ctg gct gcc tgt tta cgc gcc gat tgt tgc gag      672
   Tyr Ser Ala Ala Val Leu Ala Ala Cys Leu Arg Ala Asp Cys Cys Glu
       210                 215                 220
   att tgg acg gac gtt gac ggg gtc tat acc tgc gac ccg cgt cag gtg      720
   Ile Trp Thr Asp Val Asp Gly Val Tyr Thr Cys Asp Pro Arg Gln Val
   225                 230                 235                 240
   ccc gat gcg agg ttg ttg aag tcg atg tcc tac cag gaa gcg atg gag      768
   Pro Asp Ala Arg Leu Leu Lys Ser Met Ser Tyr Gln Glu Ala Met Glu
                   245                 250                 255
   ctt tcc tac ttc ggc gct aaa gtt ctt cac ccc cgc acc att acc ccc      816
   Leu Ser Tyr Phe Gly Ala Lys Val Leu His Pro Arg Thr Ile Thr Pro
               260                 265                 270
   atc gcc cag ttc cag atc cct tgc ctg att aaa aat acc gga aat cct      864
   Ile Ala Gln Phe Gln Ile Pro Cys Leu Ile Lys Asn Thr Gly Asn Pro
           275                 280                 285
   caa gca cca ggt acg ctc att ggt gcc agc cgt gat gaa gac gaa tta      912
   Gln Ala Pro Gly Thr Leu Ile Gly Ala Ser Arg Asp Glu Asp Glu Leu
       290                 295                 300
   ccg gtc aag ggc att tcc aat ctg aat aac atg gca atg ttc agc gtt      960
   Pro Val Lys Gly Ile Ser Asn Leu Asn Asn Met Ala Met Phe Ser Val
   305                 310                 315                 320
   tct ggt ccg ggg atg aaa ggg atg gtc ggc atg gcg gcg cgc gtc ttt     1008
   Ser Gly Pro Gly Met Lys Gly Met Val Gly Met Ala Ala Arg Val Phe
                   325                 330                 335
   gca gcg atg tca cgc gcc cgt att tcc gtg gtg ctg att acg caa tca     1056
   Ala Ala Met Ser Arg Ala Arg Ile Ser Val Val Leu Ile Thr Gln Ser
               340                 345                 350
   tct tcc gaa tac agc atc agt ttc tgc gtt cca caa agc gac tgt gtg     1104
   Ser Ser Glu Tyr Ser Ile Ser Phe Cys Val Pro Gln Ser Asp Cys Val
           355                 360                 365
   cga gct gaa cgg gca atg cag gaa gag ttc tac ctg gaa ctg aaa gaa     1152
   Arg Ala Glu Arg Ala Met Gln Glu Glu Phe Tyr Leu Glu Leu Lys Glu
       370                 375                 380
   ggc tta ctg gag ccg ctg gca gtg acg gaa cgg ctg gcc att atc tcg     1200
   Gly Leu Leu Glu Pro Leu Ala Val Thr Glu Arg Leu Ala Ile Ile Ser
   385                 390                 395                 400
   gtg gta ggt gat ggt atg cgc acc ttg cgt ggg atc tcg gcg aaa ttc     1248
   Val Val Gly Asp Gly Met Arg Thr Leu Arg Gly Ile Ser Ala Lys Phe
                   405                 410                 415
   ttt gcc gca ctg gcc cgc gcc aat atc aac att gtc gcc att gct cag     1296
   Phe Ala Ala Leu Ala Arg Ala Asn Ile Asn Ile Val Ala Ile Ala Gln
               420                 425                 430
   gga tct tct gaa cgc tca atc tct gtc gtg gta aat aac gat gat gcg     1344
   Gly Ser Ser Glu Arg Ser Ile Ser Val Val Val Asn Asn Asp Asp Ala
           435                 440                 445
   acc act ggc gtg cgc gtt act cat cag atg ctg ttc aat acc gat cag     1392
   Thr Thr Gly Val Arg Val Thr His Gln Met Leu Phe Asn Thr Asp Gln
       450                 455                 460
   gtt atc gaa gtg ttt gtg att ggc gtc ggt ggc gtt ggc ggt gcg ctg     1440
   Val Ile Glu Val Phe Val Ile Gly Val Gly Gly Val Gly Gly Ala Leu
   465                 470                 475                 480
   ctg gag caa ctg aag cgt cag caa agc tgg ctg aag aat aaa cat atc     1488
   Leu Glu Gln Leu Lys Arg Gln Gln Ser Trp Leu Lys Asn Lys His Ile
                   485                 490                 495
   gac tta cgt gtc tgc ggt gtt gcc aac tcg aag gct ctg ctc acc aat     1536
   Asp Leu Arg Val Cys Gly Val Ala Asn Ser Lys Ala Leu Leu Thr Asn
               500                 505                 510
   gta cat ggc ctt aat ctg gaa aac tgg cag gaa gaa ctg gcg caa gcc     1584
   Val His Gly Leu Asn Leu Glu Asn Trp Gln Glu Glu Leu Ala Gln Ala
           515                 520                 525
   aaa gag ccg ttt aat ctc ggg cgc tta att cgc ctc gtg aaa gaa tat     1632
   Lys Glu Pro Phe Asn Leu Gly Arg Leu Ile Arg Leu Val Lys Glu Tyr
       530                 535                 540
   cat ctg ctg aac ccg gtc att gtt gac tgc act tcc agc cag gca gtg     1680
   His Leu Leu Asn Pro Val Ile Val Asp Cys Thr Ser Ser Gln Ala Val
   545                 550                 555                 560
   gcg gat caa tat gcc gac ttc ctg cgc gaa ggt ttc cac gtt gtc acg     1728
   Ala Asp Gln Tyr Ala Asp Phe Leu Arg Glu Gly Phe His Val Val Thr
                   565                 570                 575
   ccg aac aaa aag gcc aac acc tcg tcg atg gat tac tac cat cag ttg     1776
   Pro Asn Lys Lys Ala Asn Thr Ser Ser Met Asp Tyr Tyr His Gln Leu
               580                 585                 590
   cgt tat gcg gcg gaa aaa tcg cgg cgt aaa ttc ctc tat gac acc aac     1824
   Arg Tyr Ala Ala Glu Lys Ser Arg Arg Lys Phe Leu Tyr Asp Thr Asn
           595                 600                 605
   gtt ggg gct gga tta ccg gtt att gag aac ctg caa aat ctg ctc aat     1872
   Val Gly Ala Gly Leu Pro Val Ile Glu Asn Leu Gln Asn Leu Leu Asn
       610                 615                 620
   gca ggt gat gaa ttg atg aag ttc tcc ggc att ctt tct ggt tcg ctt     1920
   Ala Gly Asp Glu Leu Met Lys Phe Ser Gly Ile Leu Ser Gly Ser Leu
   625                 630                 635                 640
   tct tat atc ttc ggc aag tta gac gaa ggc atg agt ttc tcc gag gcg     1968
   Ser Tyr Ile Phe Gly Lys Leu Asp Glu Gly Met Ser Phe Ser Glu Ala
                   645                 650                 655
   acc acg ctg gcg cgg gaa atg ggt tat acc gaa ccg gac ccg cga gat     2016
   Thr Thr Leu Ala Arg Glu Met Gly Tyr Thr Glu Pro Asp Pro Arg Asp
               660                 665                 670
   gat ctt tct ggt atg gat gtg gcg cgt aaa cta ttg att ctc gct cgt     2064
   Asp Leu Ser Gly Met Asp Val Ala Arg Lys Leu Leu Ile Leu Ala Arg
           675                 680                 685
   gaa acg gga cgt gaa ctg gag ctg gcg gat att gaa att gaa cct gtg      2112
   Glu Thr Gly Arg Glu Leu Glu Leu Ala Asp Ile Glu Ile Glu Pro Val
       690                 695                 700
   ctg ccc gca gag ttt aac gcc gag ggt gat gtt gcc gct ttt atg gcg      2160
   Leu Pro Ala Glu Phe Asn Ala Glu Gly Asp Val Ala Ala Phe Met Ala
   705                 710                 715                 720
   aat ctg tca caa ctc gac gat ctc ttt gcc gcg cgc gtg gcg aag gcc      2208
   Asn Leu Ser Gln Leu Asp Asp Leu Phe Ala Ala Arg Val Ala Lys Ala
                   725                 730                 735
   cgt gat gaa gga aaa gtt ttg cgc tat gtt ggc aat att gat gaa gat      2256
   Arg Asp Glu Gly Lys Val Leu Arg Tyr Val Gly Asn Ile Asp Glu Asp
               740                 745                 750
   ggc gtc tgc cgc gtg aag att gcc gaa gtg gat ggt aat gat ccg ctg      2304
   Gly Val Cys Arg Val Lys Ile Ala Glu Val Asp Gly Asn Asp Pro Leu
           755                 760                 765
   ttc aaa gtg aaa aat ggc gaa aac gcc ctg gcc ttc tat agc cac tat      2352
   Phe Lys Val Lys Asn Gly Glu Asn Ala Leu Ala Phe Tyr Ser His Tyr
       770                 775                 780
   tat cag ccg ctg ccg ttg gta ctg cgc gga tat ggt gcg ggc aat gac      2400
   Tyr Gln Pro Leu Pro Leu Val Leu Arg Gly Tyr Gly Ala Gly Asn Asp
   785                 790                 795                 800
   gtt aca gct gcc ggt gtc ttt gct gat ctg cta cgt acc ctc tca tgg      2448
   Val Thr Ala Ala Gly Val Phe Ala Asp Leu Leu Arg Thr Leu Ser Trp
                   805                 810                 815
   aag tta gga gtc tga                                                  2463
   Lys Leu Gly Val
               820
<210> 8
   <211> 820
   <212> PRT
   <213> Escherichia coli
<400> 8
   Met Arg Val Leu Lys Phe Gly Gly Thr Ser Val Ala Asn Ala Glu Arg
   1 5 10 15
   Phe Leu Arg Val Ala Asp Ile Leu Glu Ser Asn Ala Arg Gln Gly Gln
   20 25 30
   Val Ala Thr Val Leu Ser Ala Pro Ala Lys Ile Thr Asn His Leu Val
   35 40 45
   Ala Met Ile Glu Lys Thr Ile Ser Gly Gln Asp Ala Leu Pro Asn Ile
   50 55 60
   Ser Asp Ala Glu Arg Ile Phe Ala Glu Leu Leu Thr Gly Leu Ala Ala
   65 70 75 80
   Ala Gln Pro Gly Phe Pro Leu Ala Gln Leu Lys Thr Phe Val Asp Gln
   85 90 95
   Glu Phe Ala Gln Ile Lys His Val Leu His Gly Ile Ser Leu Leu Gly
   100 105 110
   Gln Cys Pro Asp Ser Ile Asn Ala Ala Leu Ile Cys Arg Gly Glu Lys
   115 120 125
   Met Ser Ile Ala Ile Met Ala Gly Val Leu Glu Ala Arg Gly His Asn
   130 135 140
   Val Thr Val Ile Asp Pro Val Glu Lys Leu Leu Ala Val Gly His Tyr
   145 150 155 160
   Leu Glu Ser Thr Val Asp Ile Ala Glu Ser Thr Arg Arg Ile Ala Ala
   165 170 175
   Ser Arg Ile Pro Ala Asp His Met Val Leu Met Ala Gly Phe Thr Ala
   180 185 190
   Gly Asn Glu Lys Gly Glu Leu Val Val Leu Gly Arg Asn Gly Ser Asp
   195 200 205
   Tyr Ser Ala Ala Val Leu Ala Ala Cys Leu Arg Ala Asp Cys Cys Glu
   210 215 220
   Ile Trp Thr Asp Val Asp Gly Val Tyr Thr Cys Asp Pro Arg Gln Val
   225 230 235 240
   Pro Asp Ala Arg Leu Leu Lys Ser Met Ser Tyr Gln Glu Ala Met Glu
   245 250 255
   Leu Ser Tyr Phe Gly Ala Lys Val Leu His Pro Arg Thr Ile Thr Pro
   260 265 270
   Ile Ala Gln Phe Gln Ile Pro Cys Leu Ile Lys Asn Thr Gly Asn Pro
   275 280 285
   Gln Ala Pro Gly Thr Leu Ile Gly Ala Ser Arg Asp Glu Asp Glu Leu
   290 295 300
   Pro Val Lys Gly Ile Ser Asn Leu Asn Asn Met Ala Met Phe Ser Val
   305 310 315 320
   Ser Gly Pro Gly Met Lys Gly Met Val Gly Met Ala Ala Arg Val Phe
   325 330 335
   Ala Ala Met Ser Arg Ala Arg Ile Ser Val Val Leu Ile Thr Gln Ser
   340 345 350
   Ser Ser Glu Tyr Ser Ile Ser Phe Cys Val Pro Gln Ser Asp Cys Val
   355 360 365
   Arg Ala Glu Arg Ala Met Gln Glu Glu Phe Tyr Leu Glu Leu Lys Glu
   370 375 380
   Gly Leu Leu Glu Pro Leu Ala Val Thr Glu Arg Leu Ala Ile Ile Ser
   385 390 395 400
   Val Val Gly Asp Gly Met Arg Thr Leu Arg Gly Ile Ser Ala Lys Phe
   405 410 415
   Phe Ala Ala Leu Ala Arg Ala Asn Ile Asn Ile Val Ala Ile Ala Gln
   420 425 430
   Gly Ser Ser Glu Arg Ser Ile Ser Val Val Val Asn Asn Asp Asp Ala
   435 440 445
   Thr Thr Gly Val Arg Val Thr His Gln Met Leu Phe Asn Thr Asp Gln
   450 455 460
   Val Ile Glu Val Phe Val Ile Gly Val Gly Gly Val Gly Gly Ala Leu
   465 470 475 480
   Leu Glu Gln Leu Lys Arg Gln Gln Ser Trp Leu Lys Asn Lys His Ile
   485 490 495
   Asp Leu Arg Val Cys Gly Val Ala Asn Ser Lys Ala Leu Leu Thr Asn
   500 505 510
   Val His Gly Leu Asn Leu Glu Asn Trp Gln Glu Glu Leu Ala Gln Ala
   515 520 525
   Lys Glu Pro Phe Asn Leu Gly Arg Leu Ile Arg Leu Val Lys Glu Tyr
   530 535 540
   His Leu Leu Asn Pro Val Ile Val Asp Cys Thr Ser Ser Gln Ala Val
   545 550 555 560
   Ala Asp Gln Tyr Ala Asp Phe Leu Arg Glu Gly Phe His Val Val Thr
   565 570 575
   Pro Asn Lys Lys Ala Asn Thr Ser Ser Met Asp Tyr Tyr His Gln Leu
   580 585 590
   Arg Tyr Ala Ala Glu Lys Ser Arg Arg Lys Phe Leu Tyr Asp Thr Asn
   595 600 605
   Val Gly Ala Gly Leu Pro Val Ile Glu Asn Leu Gln Asn Leu Leu Asn
   610 615 620
   Ala Gly Asp Glu Leu Met Lys Phe Ser Gly Ile Leu Ser Gly Ser Leu
   625 630 635 640
   Ser Tyr Ile Phe Gly Lys Leu Asp Glu Gly Met Ser Phe Ser Glu Ala
   645 650 655
   Thr Thr Leu Ala Arg Glu Met Gly Tyr Thr Glu Pro Asp Pro Arg Asp
   660 665 670
   Asp Leu Ser Gly Met Asp Val Ala Arg Lys Leu Leu Ile Leu Ala Arg
   675 680 685
   Glu Thr Gly Arg Glu Leu Glu Leu Ala Asp Ile Glu Ile Glu Pro Val
   690 695 700
   Leu Pro Ala Glu Phe Asn Ala Glu Gly Asp Val Ala Ala Phe Met Ala
   705 710 715 720
   Asn Leu Ser Gln Leu Asp Asp Leu Phe Ala Ala Arg Val Ala Lys Ala
   725 730 735
   Arg Asp Glu Gly Lys Val Leu Arg Tyr Val Gly Asn Ile Asp Glu Asp
   740 745 750
   Gly Val Cys Arg Val Lys Ile Ala Glu Val Asp Gly Asn Asp Pro Leu
   755 760 765
   Phe Lys Val Lys Asn Gly Glu Asn Ala Leu Ala Phe Tyr Ser His Tyr
   770 775 780
   Tyr Gln Pro Leu Pro Leu Val Leu Arg Gly Tyr Gly Ala Gly Asn Asp
   785 790 795 800
   Val Thr Ala Ala Gly Val Phe Ala Asp Leu Leu Arg Thr Leu Ser Trp
   805 810 815
   Lys Leu Gly Val
   820
<210> 9
   <211> 2460
   <212> DNA
   <213> Serratia marcescens
<220>
   <221> CDS
   <222> (1)..(2460)
   <223> thrA-Kodierregion
<400> 9
   atg cga gtg ctg aaa ttt ggc gga acc tcg gta gcg aat gcg gaa cgt       48
   Met Arg Val Leu Lys Phe Gly Gly Thr Ser Val Ala Asn Ala Glu Arg
   1               5                   10                  15
   ttt ctg cgc gtc gcc gac atc atg gag agt aac gcg cgt cag gga cag       96
   Phe Leu Arg Val Ala Asp Ile Met Glu Ser Asn Ala Arg Gln Gly Gln
               20                  25                  30
   gta gcc acg gtc ttg tcc gcc ccc gca aag atc acc aac cat ctg gtg      144
   Val Ala Thr Val Leu Ser Ala Pro Ala Lys Ile Thr Asn His Leu Val
           35                  40                  45
   gcg atg atc gac aaa acg gtg gcg ggc cag gac att ctg ccg aat atg      192
   Ala Met Ile Asp Lys Thr Val Ala Gly Gln Asp Ile Leu Pro Asn Met
       50                  55                  60
   agc gac gcc gag cgg atc ttt gcc gac ctg ctg agc gga ctg gcg cag      240
   Ser Asp Ala Glu Arg Ile Phe Ala Asp Leu Leu Ser Gly Leu Ala Gln
   65                  70                  75                  80
   gcg ctg ccg ggc ttt gaa tac gat cgt ttg aaa ggc gtg gtc gat cag      288
   Ala Leu Pro Gly Phe Glu Tyr Asp Arg Leu Lys Gly Val Val Asp Gln
                   85                  90                  95
   gaa ttc gcg cag ctc aaa cag gtg ctg cat ggc gtc tcg ctg ctg ggg      336
   Glu Phe Ala Gln Leu Lys Gln Val Leu His Gly Val Ser Leu Leu Gly
               100                 105                 110
   cag tgc ccg gac agc gtg aac gcg gcg atc atc tgc cgt ggc gaa aag      384
   Gln Cys Pro Asp Ser Val Asn Ala Ala Ile Ile Cys Arg Gly Glu Lys
           115                 120                 125
   ctc tcc atc gcc atc atg gaa ggg gtg ttc cgc gcc aag ggt tat ccg      432
   Leu Ser Ile Ala Ile Met Glu Gly Val Phe Arg Ala Lys Gly Tyr Pro
       130                 135                 140
   gtc acg gtg atc aac ccg gtg gag aaa ctg ctg gcg cag ggc cac tac      480
   Val Thr Val Ile Asn Pro Val Glu Lys Leu Leu Ala Gln Gly His Tyr
   145                 150                 155                 160
   ctg gag tcc acc gtg gac atc gcc gag tct acg ctg cgc atc gcc gcc      528
   Leu Glu Ser Thr Val Asp Ile Ala Glu Ser Thr Leu Arg Ile Ala Ala
                   165                 170                 175
   gcg gcg atc ccg gcc gat cac atc gtg ctg atg gcc ggt ttc acc gcc      576
   Ala Ala Ile Pro Ala Asp His Ile Val Leu Met Ala Gly Phe Thr Ala
               180                 185                 190
   ggt aat gat aag ggc gag ctg gtg gtg ctg ggc cgc aac ggc tcc gac      624
   Gly Asn Asp Lys Gly Glu Leu Val Val Leu Gly Arg Asn Gly Ser Asp
           195                 200                 205
   tat tcc gcc gcg gtg ctg gcc gcc tgc ctg cgc gcc gac tgt tgc gag      672
   Tyr Ser Ala Ala Val Leu Ala Ala Cys Leu Arg Ala Asp Cys Cys Glu
       210                 215                 220
   atc tgg acc gac gtc gac ggc gtt tat acc tgc gat ccg cgc acc gtg      720
   Ile Trp Thr Asp Val Asp Gly Val Tyr Thr Cys Asp Pro Arg Thr Val
   225                 230                 235                 240
   ccg gac gcc agg tta ctg aaa tcg atg tcg tac cag gaa gcg atg gag      768
   Pro Asp Ala Arg Leu Leu Lys Ser Met Ser Tyr Gln Glu Ala Met Glu
                   245                 250                 255
   ctt tcc tat ttc ggc gcc aaa gtg tta cac ccc cgc acc atc act ccg      816
   Leu Ser Tyr Phe Gly Ala Lys Val Leu His Pro Arg Thr Ile Thr Pro
               260                 265                 270
   att gcc cag ttt caa atc cct tgc ctg att aaa aac acc tcc aac ccg      864
   Ile Ala Gln Phe Gln Ile Pro Cys Leu Ile Lys Asn Thr Ser Asn Pro
           275                 280                 285
   cag gcc ccc ggc acg ctg atc ggc aaa gac agc acc gat gcc gat atg      912
   Gln Ala Pro Gly Thr Leu Ile Gly Lys Asp Ser Thr Asp Ala Asp Met
       290                 295                 300
   ccg gtg aag ggc atc acc aac ctg aac aac atg gcg atg atc aac gtc      960
   Pro Val Lys Gly Ile Thr Asn Leu Asn Asn Met Ala Met Ile Asn Val
   305                 310                 315                 320
   tcc ggc ccg ggc atg aaa ggc atg gtc ggc atg gcc gca cgg gtc ttc     1008
   Ser Gly Pro Gly Met Lys Gly Met Val Gly Met Ala Ala Arg Val Phe
                   325                 330                 335
   gcc gtg atg tcg cgc gcc ggc att tcg gtg gtg ctg atc acc caa tcc     1056
   Ala Val Met Ser Arg Ala Gly Ile Ser Val Val Leu Ile Thr Gln Ser
               340                 345                 350
   tct tcc gaa tac agc atc agc ttc tgc gtg ccg cag ggt gaa ctg cag     1104
   Ser Ser Glu Tyr Ser Ile Ser Phe Cys Val Pro Gln Gly Glu Leu Gln
           355                 360                 365
   cgc gcc cgt cgc gcg ctg gaa gag gag ttc tat ctg gag ctg aaa gac     1152
   Arg Ala Arg Arg Ala Leu Glu Glu Glu Phe Tyr Leu Glu Leu Lys Asp
       370                 375                 380
   ggc gtg ctg gat ccg ctg gac gtg atg gaa cgc ctg gcg atc atc tcg      1200
   Gly Val Leu Asp Pro Leu Asp Val Met Glu Arg Leu Ala Ile Ile Ser
   385                 390                 395                 400
   gtg gtt ggc gac ggc atg cgc acc ctg cgc ggc att tcc gca cgc ttc      1248
   Val Val Gly Asp Gly Met Arg Thr Leu Arg Gly Ile Ser Ala Arg Phe
                   405                 410                 415
   ttc tcc gcg ctg gcg cgc gcc aac atc aat atc gtt gcc atc gcc caa      1296
   Phe Ser Ala Leu Ala Arg Ala Asn Ile Asn Ile Val Ala Ile Ala Gln
               420                 425                 430
   ggg tct tcc gaa cgt tcc att tcg gtg gtg gtc agc aat gat tcc gcc      1344
   Gly Ser Ser Glu Arg Ser Ile Ser Val Val Val Ser Asn Asp Ser Ala
           435                 440                 445
   acc acc ggc gtg cgc gtc agc cac cag atg ctg ttc aac acc gat cag      1392
   Thr Thr Gly Val Arg Val Ser His Gln Met Leu Phe Asn Thr Asp Gln
       450                 455                 460
   gtg atc gaa gtg ttc gtc atc ggc gtc ggc ggc gtc ggc ggg gcg ctg      1440
   Val Ile Glu Val Phe Val Ile Gly Val Gly Gly Val Gly Gly Ala Leu
   465                 470                 475                 480
   atc gag cag atc tat cgc cag cag ccg tgg ctg aag caa aaa cat atc      1488
   Ile Glu Gln Ile Tyr Arg Gln Gln Pro Trp Leu Lys Gln Lys His Ile
                   485                 490                 495
   gat ctg cgg gtg tgc ggc atc gcc aac tcg cgc gtg atg ctg acc aat      1536
   Asp Leu Arg Val Cys Gly Ile Ala Asn Ser Arg Val Met Leu Thr Asn
               500                 505                 510
   gtg cac ggc atc gcg ctg gac agc tgg cgc gac gcg ctg gcc ggc gcg      1584
   Val His Gly Ile Ala Leu Asp Ser Trp Arg Asp Ala Leu Ala Gly Ala
           515                 520                 525
   cag gag ccg ttc aat ctc ggc cgt ctg atc cgg ctg gtg aag gaa tat      1632
   Gln Glu Pro Phe Asn Leu Gly Arg Leu Ile Arg Leu Val Lys Glu Tyr
       530                 535                 540
   cac ctg ctg aac ccg gtg atc gtc gac tgt acc tcc agt cag gcg gtg      1680
   His Leu Leu Asn Pro Val Ile Val Asp Cys Thr Ser Ser Gln Ala Val
   545                 550                 555                 560
   gcc gat cag tac gtg gac ttc ctg gcg gac ggc ttc cat gtg gtg acg      1728
   Ala Asp Gln Tyr Val Asp Phe Leu Ala Asp Gly Phe His Val Val Thr
                   565                 570                 575
   ccg aac aaa aag gcc aac acc tcg tcg atg aac tat tat cag caa ctg      1776
   Pro Asn Lys Lys Ala Asn Thr Ser Ser Met Asn Tyr Tyr Gln Gln Leu
               580                 585                 590
   cgc gcg gcc gcc gcc ggt tcg cat cgc aag ttc ctg tac gac acc aac      1824
   Arg Ala Ala Ala Ala Gly Ser His Arg Lys Phe Leu Tyr Asp Thr Asn
           595                 600                 605
   gtc ggt gcc ggt ctg ccg gtg att gag aac ctg caa aac ctg ctg aac      1872
   Val Gly Ala Gly Leu Pro Val Ile Glu Asn Leu Gln Asn Leu Leu Asn
       610                 615                 620
   gcc ggt gat gaa ctg gta cgt ttc tcc ggt atc ctg tcc ggc tcg ctg     1920
   Ala Gly Asp Glu Leu Val Arg Phe Ser Gly Ile Leu Ser Gly Ser Leu
   625                 630                 635                 640
   tcc ttt atc ttc ggc aag ctg gac gaa ggg ctg tcg ctg tcg gcg gcg     1968
   Ser Phe Ile Phe Gly Lys Leu Asp Glu Gly Leu Ser Leu Ser Ala Ala
                   645                 650                 655
   acc ctg cag gcc cgg gcc aac ggc tac acc gag ccg gat ccg cgc gac     2016
   Thr Leu Gln Ala Arg Ala Asn Gly Tyr Thr Glu Pro Asp Pro Arg Asp
               660                 665                 670
   gat ctg tcc ggg atg gat gtg gcg cgt aag ctg ctg atc ctg gcg cgc     2064
   Asp Leu Ser Gly Met Asp Val Ala Arg Lys Leu Leu Ile Leu Ala Arg
           675                 680                 685
   gag gcc ggt tac aaa ctg gaa ctg agc gat atc gag gtc gag ccg gtg     2112
   Glu Ala Gly Tyr Lys Leu Glu Leu Ser Asp Ile Glu Val Glu Pro Val
       690                 695                 700
   ctg ccg ccg tcc ttc gac gcg tcg ggc gac gtg gac acc ttc ctg gcg     2160
   Leu Pro Pro Ser Phe Asp Ala Ser Gly Asp Val Asp Thr Phe Leu Ala
   705                 710                 715                 720
   cgg ttg ccg gag ctg gat aaa gag ttc gcg cgc aac gtg gct aac gcc     2208
   Arg Leu Pro Glu Leu Asp Lys Glu Phe Ala Arg Asn Val Ala Asn Ala
                   725                 730                 735
   gcc gag caa ggc aag gtg ctg cgc tat gtc ggc ctg atc gac gaa ggg     2256
   Ala Glu Gln Gly Lys Val Leu Arg Tyr Val Gly Leu Ile Asp Glu Gly
               740                 745                 750
   cgc tgc aag gtg cgc att gag gcg gtg gac ggc aac gat ccg ttg tat     2304
   Arg Cys Lys Val Arg Ile Glu Ala Val Asp Gly Asn Asp Pro Leu Tyr
           755                 760                 765
   aaa gtg aag aac ggc gag aac gcg ctg gcc ttc tac agc cgc tac tat     2352
   Lys Val Lys Asn Gly Glu Asn Ala Leu Ala Phe Tyr Ser Arg Tyr Tyr
       770                 775                 780
   cag ccg ttg ccg ctg gtg ctg cgc ggc tac ggc gcc ggt aac gat gtg     2400
   Gln Pro Leu Pro Leu Val Leu Arg Gly Tyr Gly Ala Gly Asn Asp Val
   785                 790                 795                 800
   act gca gcg ggc gta ttc gcc gat ctg ctg cgc aca ctg tca tgg aag     2448
   Thr Ala Ala Gly Val Phe Ala Asp Leu Leu Arg Thr Leu Ser Trp Lys
                   805                 810                 815
   ttg gga gtt taa                                                     2460
   Leu Gly Val
<210> 10
   <211> 819
   <212> PRT
   <213> Serratia marcescens
<400> 10
   Met Arg Val Leu Lys Phe Gly Gly Thr Ser Val Ala Asn Ala Glu Arg
   1               5                   10                  15
   Phe Leu Arg Val Ala Asp Ile Met Glu Ser Asn Ala Arg Gln Gly Gln
               20                  25                  30
   Val Ala Thr Val Leu Ser Ala Pro Ala Lys Ile Thr Asn His Leu Val
           35                  40                  45
   Ala Met Ile Asp Lys Thr Val Ala Gly Gln Asp Ile Leu Pro Asn Met
       50                  55                  60
   Ser Asp Ala Glu Arg Ile Phe Ala Asp Leu Leu Ser Gly Leu Ala Gln
   65                  70                  75                  80
   Ala Leu Pro Gly Phe Glu Tyr Asp Arg Leu Lys Gly Val Val Asp Gln
                   85                  90                  95
   Glu Phe Ala Gln Leu Lys Gln Val Leu His Gly Val Ser Leu Leu Gly
               100                 105                 110
   Gln Cys Pro Asp Ser Val Asn Ala Ala Ile Ile Cys Arg Gly Glu Lys
           115                 120                 125
   Leu Ser Ile Ala Ile Met Glu Gly Val Phe Arg Ala Lys Gly Tyr Pro
       130                 135                 140
   Val Thr Val Ile Asn Pro Val Glu Lys Leu Leu Ala Gln Gly His Tyr
   145                 150                 155                 160
   Leu Glu Ser Thr Val Asp Ile Ala Glu Ser Thr Leu Arg Ile Ala Ala
                   165                 170                 175
   Ala Ala Ile Pro Ala Asp His Ile Val Leu Met Ala Gly Phe Thr Ala
               180                 185                 190
   Gly Asn Asp Lys Gly Glu Leu Val Val Leu Gly Arg Asn Gly Ser Asp
           195                 200                 205
   Tyr Ser Ala Ala Val Leu Ala Ala Cys Leu Arg Ala Asp Cys Cys Glu
       210                 215                 220
   Ile Trp Thr Asp Val Asp Gly Val Tyr Thr Cys Asp Pro Arg Thr Val
   225                 230                 235                 240
   Pro Asp Ala Arg Leu Leu Lys Ser Met Ser Tyr Gln Glu Ala Met Glu
                   245                 250                 255
   Leu Ser Tyr Phe Gly Ala Lys Val Leu His Pro Arg Thr Ile Thr Pro
               260                 265                 270
   Ile Ala Gln Phe Gln Ile Pro Cys Leu Ile Lys Asn Thr Ser Asn Pro
           275                 280                 285
   Gln Ala Pro Gly Thr Leu Ile Gly Lys Asp Ser Thr Asp Ala Asp Met
       290                 295                 300
   Pro Val Lys Gly Ile Thr Asn Leu Asn Asn Met Ala Met Ile Asn Val
   305                 310                 315                 320
   Ser Gly Pro Gly Met Lys Gly Met Val Gly Met Ala Ala Arg Val Phe
                   325                 330                 335
   Ala Val Met Ser Arg Ala Gly Ile Ser Val Val Leu Ile Thr Gln Ser
               340                 345                 350
   Ser Ser Glu Tyr Ser Ile Ser Phe Cys Val Pro Gln Gly Glu Leu Gln
           355                 360                 365
   Arg Ala Arg Arg Ala Leu Glu Glu Glu Phe Tyr Leu Glu Leu Lys Asp
       370                 375                 380
   Gly Val Leu Asp Pro Leu Asp Val Met Glu Arg Leu Ala Ile Ile Ser
   385                 390                 395                 400
   Val Val Gly Asp Gly Met Arg Thr Leu Arg Gly Ile Ser Ala Arg Phe
                   405                 410                 415
   Phe Ser Ala Leu Ala Arg Ala Asn Ile Asn Ile Val Ala Ile Ala Gln
               420                 425                 430
   Gly Ser Ser Glu Arg Ser Ile Ser Val Val Val Ser Asn Asp Ser Ala
           435                 440                 445
   Thr Thr Gly Val Arg Val Ser His Gln Met Leu Phe Asn Thr Asp Gln
       450                 455                 460
   Val Ile Glu Val Phe Val Ile Gly Val Gly Gly Val Gly Gly Ala Leu
   465                 470                 475                 480
   Ile Glu Gln Ile Tyr Arg Gln Gln Pro Trp Leu Lys Gln Lys His Ile
                   485                 490                 495
   Asp Leu Arg Val Cys Gly Ile Ala Asn Ser Arg Val Met Leu Thr Asn
               500                 505                 510
   Val His Gly Ile Ala Leu Asp Ser Trp Arg Asp Ala Leu Ala Gly Ala
           515                 520                 525
   Gln Glu Pro Phe Asn Leu Gly Arg Leu Ile Arg Leu Val Lys Glu Tyr
       530                 535                 540
   His Leu Leu Asn Pro Val Ile Val Asp Cys Thr Ser Ser Gln Ala Val
   545                 550                 555                 560
   Ala Asp Gln Tyr Val Asp Phe Leu Ala Asp Gly Phe His Val Val Thr
                   565                 570                 575
   Pro Asn Lys Lys Ala Asn Thr Ser Ser Met Asn Tyr Tyr Gln Gln Leu
               580                 585                 590
   Arg Ala Ala Ala Ala Gly Ser His Arg Lys Phe Leu Tyr Asp Thr Asn
           595                 600                 605
   Val Gly Ala Gly Leu Pro Val Ile Glu Asn Leu Gln Asn Leu Leu Asn
       610                 615                 620
   Ala Gly Asp Glu Leu Val Arg Phe Ser Gly Ile Leu Ser Gly Ser Leu
   625                 630                 635                 640
   Ser Phe Ile Phe Gly Lys Leu Asp Glu Gly Leu Ser Leu Ser Ala Ala
                   645                 650                 655
   Thr Leu Gln Ala Arg Ala Asn Gly Tyr Thr Glu Pro Asp Pro Arg Asp
               660                 665                 670
   Asp Leu Ser Gly Met Asp Val Ala Arg Lys Leu Leu Ile Leu Ala Arg
           675                 680                 685
   Glu Ala Gly Tyr Lys Leu Glu Leu Ser Asp Ile Glu Val Glu Pro Val
       690                 695                 700
   Leu Pro Pro Ser Phe Asp Ala Ser Gly Asp Val Asp Thr Phe Leu Ala
   705                 710                 715                 720
   Arg Leu Pro Glu Leu Asp Lys Glu Phe Ala Arg Asn Val Ala Asn Ala
   725 730 735
   Ala Glu Gln Gly Lys Val Leu Arg Tyr Val Gly Leu Ile Asp Glu Gly
   740 745 750
   Arg Cys Lys Val Arg Ile Glu Ala Val Asp Gly Asn Asp Pro Leu Tyr
   755 760 765
   Lys Val Lys Asn Gly Glu Asn Ala Leu Ala Phe Tyr Ser Arg Tyr Tyr
   770 775 780
   Gln Pro Leu Pro Leu Val Leu Arg Gly Tyr Gly Ala Gly Asn Asp Val
   785 790 795 800
   Thr Ala Ala Gly Val Phe Ala Asp Leu Leu Arg Thr Leu Ser Trp Lys
   805 810 815
   Leu Gly Val
<210> 11
   <211> 26
   <212> DNA
   <213> Escherichia coli
<220>
   <221> misc_feature
   <222> (1).. (26)
   <223> uspA-1 Primer
<400> 11
   tctagagtga cgtaacggca caagaa 26
<210> 12
   <211> 26
   <212> DNA
   <213> Escherichia coli
<220>
   <221> misc_feature
   <222> (1).. (26)
   <223> uspA-2 Primer
<400> 12
   aagcttttca ggaacacgtc gtagag 26
<210> 13
   <211> 539
   <212> DNA
   <213> Escherichia coli
<220>
   <221> CDS
   <222> (73)..(507)
   <223> uspA-Kodierregion
<400> 13
   tctagagtga cgtaacggca caagaaacgc tagctggcca gtcatcgaca actttatgga      60
   aggagtaaca ct atg gct tat aaa cac att ctc atc gcg gtc gac ctc tcc     111
                 Met Ala Tyr Lys His Ile Leu Ile Ala Val Asp Leu Ser
                 1               5                   10
   ccg gaa agc aaa gtt ctg gta gag aaa gca gtc tct atg gct cgc ccc       159
   Pro Glu Ser Lys Val Leu Val Glu Lys Ala Val Ser Met Ala Arg Pro
       15                  20                  25
   tac aat gcg aaa gtt tct ctg atc cac gta gat gta aac tac tct gac       207
   Tyr Asn Ala Lys Val Ser Leu Ile His Val Asp Val Asn Tyr Ser Asp
   30                  35                  40                  45
   cta tac acc ggg ctt att gat gtg aat ctg ggt gat atg cag aaa cgc       255
   Leu Tyr Thr Gly Leu Ile Asp Val Asn Leu Gly Asp Met Gln Lys Arg
                   50                  55                  60
   atc tct gaa gag aca cat cat gca ctg acc gag ctt tcc act aat gca       303
   Ile Ser Glu Glu Thr His His Ala Leu Thr Glu Leu Ser Thr Asn Ala
               65                  70                  75
   ggc tac cca atc act gaa acc ctg agc ggc agc ggc gac ctg ggc cag       351
   Gly Tyr Pro Ile Thr Glu Thr Leu Ser Gly Ser Gly Asp Leu Gly Gln
           80                  85                  90
   gtt ctg gtc gat gca atc aag aaa tac gat atg gat ttg gtg gtt tgt       399
   Val Leu Val Asp Ala Ile Lys Lys Tyr Asp Met Asp Leu Val Val Cys
       95                  100                 105
   ggt cac cac cag gac ttc tgg agc aaa ctg atg tct tcc gca cgt cag       447
   Gly His His Gln Asp Phe Trp Ser Lys Leu Met Ser Ser Ala Arg Gln
   110                 115                 120                 125
   ctg atc aac acc gtt cac gtt gat atg ctg att gtt ccg ctc gcg gac       495
   Leu Ile Asn Thr Val His Val Asp Met Leu Ile Val Pro Leu Ala Asp
                   130                 135                 140
   gaa gaa gaa taa tcttccctct acgacgtgtt cctgaaaagc tt                   539
   Glu Glu Glu
<210> 14
   <211> 144
   <212> PRT
   <213> Escherichia coli
<400> 14
   Met Ala Tyr Lys His Ile Leu Ile Ala Val Asp Leu Ser Pro Glu Ser
   1               5                   10                  15
   Lys Val Leu Val Glu Lys Ala Val Ser Met Ala Arg Pro Tyr Asn Ala
               20                  25                  30
   Lys Val Ser Leu Ile His Val Asp Val Asn Tyr Ser Asp Leu Tyr Thr
           35                  40                  45
   Gly Leu Ile Asp Val Asn Leu Gly Asp Met Gln Lys Arg Ile Ser Glu
       50                  55                  60
   Glu Thr His His Ala Leu Thr Glu Leu Ser Thr Asn Ala Gly Tyr Pro
   65                  70                  75                  80
   Ile Thr Glu Thr Leu Ser Gly Ser Gly Asp Leu Gly Gln Val Leu Val
                   85                  90                  95
   Asp Ala Ile Lys Lys Tyr Asp Met Asp Leu Val Val Cys Gly His His
               100                 105                 110
   Gln Asp Phe Trp Ser Lys Leu Met Ser Ser Ala Arg Gln Leu Ile Asn
           115                 120                 125
   Thr Val His Val Asp Met Leu Ile Val Pro Leu Ala Asp Glu Glu Glu
       130                 135                 140

## Beschreibung

Diese Erfindung betrifft ein Verfahren zur fermentativen Herstellung von L-Aminosäuren, insbesondere L-Threonin, L-Tryptophan, L-Lysin, L-Valin, L-Isoleucin und L-Homoserin, unter Verwendung von rekombinanten Mikroorganismen der Familie Enterobacteriaceae, in denen das uspA-Gen, welches für das universelle Stressprotein A kodiert, verstärkt, insbesondere überexprimiert wird, und diese Mikroorganismen.

### Stand der Technik

L-Aminosäuren, insbesondere L-Threonin, L-Tryptophan, L-Lysin, L-Valin, L-Isoleucin und L-Homoserin, finden in der Humanmedizin und in der pharmazeutischen Industrie, in der Lebensmittelindustrie und in der Tierernährung Anwendung.

Es ist bekannt, dass L-Aminosäuren durch Fermentation von Stämmen der Enterobacteriaceae, insbesondere Escherichia coli (E. coli) und Serratia marcescens, hergestellt werden. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie zum Beispiel die Auswahl des verwendeten Zuckers oder die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

In Wildtypstämmen verhindern strikte Regulationsmechanismen die über den Eigenbedarf hinausgehende Produktion von Stoffwechselprodukten wie Aminosäuren und deren Abgabe ins Medium. Die Konstruktion von aus Herstellersicht Aminosäure überproduzierenden Stämmen erfordert deshalb, diese Stoffwechselregulationen zu überwinden.

Zur Beseitigung der Kontrollmechanismen und Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren. Resistenz gegen das Tryptophan-Analogon 5-Methyl-DL-Tryptophan (5-MT) zeichnet zum Beispiel einen Stamm aus, der L-Tryptophan produziert.

Seit einigen Jahren werden ebenfalls Methoden der rekombinanten DNA-Technik zur gezielten Stammverbesserung L-Aminosäuren produzierender Stämme der Familie Enterobacteriaceae eingesetzt, indem man zum Beispiel einzelne Aminosäure-Biosynthesegene amplifiziert oder die Eigenschaften spezieller Gene verändert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2nd edition, ASM Press, Washington, D.C., USA, (1996) zu finden. Eine Zusammenfassung zum Stoffwechsel und zur Produktion von L-Threonin ist veröffentlicht durch Debabov (Advances in Biochemical Engineering Vol.79, 113-136 (2003)).

Das uspA-Gen kodiert für das zytoplasmatische universelle Stressprotein A, einem Vertreter der Usp-Proteinfamilie, das verstärkt während längerer Phasen kompletter Wachstumsinhibierung exprimiert wird. Deletionen in uspA führen dabei zu einer Erniedrigung der Überlebensrate nach Behandlung von Zellen mit 1 M NaCl (osmotischer Stress), CdCl₂, H₂O₂, DNP (=DinitrophenoI) und CCCP (=Carbonylcyanid-m-chlor-phenylhydrazon) (Nyström und Neidhardt, Molecular Microbiology 11(3): 534-544 (1994)). Für die Gene uspA, uspC, uspD und uspE der Usp-Proteinfamilie konnte nachgewiesen werden, dass deren Genprodukte eine kooperative Schutzfunktion gegenüber UV-Schäden ausüben (Gustavsson et al., Molecular Microbiology 43:107-117 (2002)). Das UspA Protein wird unter unterschiedlichsten Bedingungen, wie bei Nährstofflimitation von Kohlenstoff, Stickstoff, Phosphat, Schwefel und Aminosäuren, bei dem Eintritt in die stationäre Phase, sowie bei moderater Hitze (bis 42°C), Ethanolstress (4%), osmotischem Stress, und in Anwesenheit verschiedenster toxischer Substanzen (Mitomycin C, H₂O₂, CCCP, DNP, und weitere), verstärkt synthetisiert (Nystrom und Neidhardt, Molecular Microbiology 6(21): 3187-3198 (1992); Diez et al., Molecular Microbiology 36(6): 1494-1503 (2000)) und an Serin/Threonin-Aminosäureresten autophosphoryliert (Freestone et al., Journal of Molecular Microbiology 274(3): 318-324 (1997)). Gleiche Bedingungen führten ebenfalls zu einer verstärkten Synthese der Usp-Proteine UspC, UspD und UspE in E. coli, diese werden somit koordiniert reguliert (Nachin et al., Journal of Bacteriology 187(18): 6265-6272 (2005)). Dabei scheint das Alarmon ppGpp essentiell für eine Induktion von uspA, uspC, uspD, uspE zu sein (Gustavsson et al., Molecular Microbiology 43:107-117 (2002); Kvint et al., Current Opinion in Microbiology 6(2): 140-145 (2003)). Zudem stehen die Gene uspA und uspD unter der negativen Kontrolle des FadR-Regulators, der bei der Regulation des Fettsäure-Metabolismus in E coli eine wichtige Rolle spielt (Kvint et al., Current Opinion in Microbiology 6(2): 140-145 (2003); Farewell et al., Journal of Bacteriology 178(22): 6443-6450 (1996)). Während der stationären Phase wird uspA induziert, indem durch das Alarmon ppGpp über eine stringente RNA-Polymerase die negative Regulation von uspA durch den FadR-Repressor aufgehoben wird (Kvint et al., Molecular Microbiology 35(2): 435-443 (2000)). Die Überexpression von uspA führt zu einer verringerten Wachstumsrate in Minimalmedium und verursacht weitreichende Änderungen im Protein-Synthese-Muster (Nystrom und Neidhardt, Journal of Bacteriology 178(3): 927-930 (1996)).

Die Nukleotidsequenz der Wildform des für das universelle Stressprotein A von Escherichia coli kodierenden Gens ist in der Datenbank des National Center for Biotechnology Information (NCBI) der National Library of Medicin (Bethesda, MD, USA) unter der Zugangsnummer (accession number) NC_000913 (Region: 3638134-3638568) allgemein verfügbar.

### Aufgabe der Erfindung

Die Erfinder haben sich die Aufgabe gestellt, neue Maßnahmen zur Herstellung erhöhter Mengen von L-Aminosäuren, insbesondere L-Threonin, L-Tryptophan, L-Lysin, L-Valin, L-Isoleucin und L-Homoserin, bereitzustellen.

### Beschreibung der Erfindung

Gegenstand der Erfindung ist ein Verfahren zur Herstellung von L-Aminosäuren, insbesondere von L-Threonin, L-Tryptophan, L-Lysin, L-Valin, L-Isoleucin und L-Homoserin, durch Fermentation von rekombinanten Mikroorganismen der Familie Enterobacteriaceae, die ein überexprimiertes uspA-Gen enthalten, welches für das universelle Stressprotein A kodiert, dadurch gekennzeichnet, dass man
a) die die gewünschte L-Aminosäure produzierenden Mikroorganismen, in denen man das uspA-Gen oder für dessen Genprodukt kodierende Nukleotidsequenzen oder Allele überexprimiert, in einem Medium kultiviert unter Bedingungen, bei denen die gewünschte L-Aminosäure im Medium oder in den Zellen angereichert wird, und
b) die gewünschte L-Aminosäure isoliert, wobei gegebenenfalls weitere Bestandteile der Fermentationsbrühe und/oder die Biomasse in ihrer Gesamtheit oder Anteilen (≥ 0 bis 100 %) im isolierten Produkt verbleiben oder vollständig entfernt werden, wobei in den zur Herstellung von L-Aminosäuren fermentierten Mikroorganismen die Überexpression des uspA-Gens dadurch erzielt wird, dass man einen starken Promotor funktionell mit dem uspA-Gen verknüpft.

In dem erfindungsgemäßen Verfahren eingesetzt werden entsprechend rekombinante L-Aminosäure produzierende Mikroorganismen der Familie Enterobacteriaceae, die ein überexprimiertes uspA-Gen, das für das universelle Stressprotein A kodiert, oder für dessen Genprodukt kodierende Nukleotidsequenzen enthalten, und die vermehrt L-Aminosäuren, insbesondere L-Threonin, L-Tryptophan, L-Lysin, L-Valin, L-Isoleucin und L-Homoserin, produzieren und in der Zelle oder im Medium anreichern.

Als Ausgangspunkt für den Vergleich dienen jeweils die für das uspA-Gen nicht rekombinanten Mikroorganismen, die kein verstärktes uspA-Gen enthalten und an denen die erfindungsgemäße Überexpression nicht durchgeführt wurde.

Zu den erfindungsgemäß eingesetzten Mikroorganismen (oder Ausgangs- Mikroorganismen) gehören insbesondere Mikroorganismen der Familie Enterobacteriaceae, in denen ein Polynukleotid verstärkt wird, das für ein Polypeptid kodiert, dessen Aminosäuresequenz zu mindestens 80% oder zu mindestens 90%, insbesondere zu mindestens 95%, bevorzugt zu mindestens 98% oder mindestens 99%, besonders bevorzugt zu 99,6% und ganz besonders bevorzugt zu 100% identisch ist mit einer Aminosäuresequenz, ausgewählt aus der Gruppe SEQ ID No. 2, SEQ ID No. 4 und SEQ ID No. 6, bevorzugt SEQ ID No. 2, wobei das Polypeptid die Aktivität des universellen Stressproteins A besitzt.

Die erfindungsgemäß eingesetzten Mikroorganismen enthalten bevorzugt dem uspA-Gen entsprechende isolierte Polynukleotide, ausgewählt aus der Gruppe:
a) Polynukleotid mit einer Nukleotidsequenz, ausgewählt aus SEQ ID No. 1, SEQ ID No. 3, SEQ ID No. 5 und den dazu komplementären Nukleotidsequenzen;
b) Polynukleotid mit einer Nukleotidsequenz, die der SEQ ID No. 1, SEQ ID No. 3 oder SEQ ID No. 5 im Rahmen der Degeneration des genetischen Codes entspricht;
c) Polynukleotidsequenz mit einer Sequenz, die mit der zur Sequenz SEQ ID No. 1, SEQ ID No. 3 oder SEQ ID No. 5 komplementären Sequenz unter stringenten Bedingungen hybridisiert, wobei die stringenten Bedingungen bevorzugt durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1, SEQ ID No. 3 oder SEQ ID No. 5, die funktionsneutrale Sinnmutanten enthält,
wobei die Polynukleotide für das universelle Stressprotein A kodieren.

Die erfindungsgemäß eingesetzten rekombinanten Mikroorganismen der Familie Enterobacteriaceae produzieren vorzugsweise bereits vor der erfindungsgemäßen Verstärkung oder Überexpression L-Aminosäuren.

Als vor der erfindungsgemäßen Verstärkung oder Überexpression L-Aminosäure produzierende Mikroorganismen zählen dabei nicht die Wildtypstämme und häufig benutzte Laborstämme wie unter anderen DH5α, DH5αmcr, W3110, JM105, MG1655, MC4100, Y1089, H560 und MM152.

Bevorzugt werden die beschriebenen Mikroorganismen eingesetzt.

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin gemeint. Besonders bevorzugt sind L-Threonin, L-Tryptophan, L-Lysin, L-Valin, L-Isoleucin und L-Homoserin.

Der Begriff "Verstärkung" beschreibt erfindungsgemäß die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs um mindestens eine (1) Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die erfindungsgemäße Verstärkung oder Überexpression durchgeführt wird.

Die Konzentration des Proteins kann über 1- und 2-dimensionale Proteingelauftrennung und anschließende optische Identifizierung der Proteinkonzentration mit enstprechender Auswertesoftware im Gel bestimmt werden. Gebräuchliche Methoden zur Präparation der Proteingele und zur Identifizierung der Proteine sind die im 2-D Electrophoresis Handbook (GE Healthcare UK Ltd, England, Handbook 80-6429-60AC (2006)) und die von Hermann et al. (Electrophoresis, 22:1712-23 (2001))beschriebenen Vorgehensweisen. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich, Angewandte Chemie 38: 2630-2647 (1999)) bestimmt werden.

Ein Gen oder Allel ist chemisch ein Polynukleotid. Ein anderer Begriff hierfür ist Nukleinsäure, insbesondere Desoxyribonukleinsäure.

Die Begriff Polypeptid und Protein sind gegenseitig austauschbar.

Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure.

Die insbesondere rekombinanten Mikroorganismen mit einem verstärkten oder überexprimierten uspA-Gen können L-Aminosäuren aus Glucose, Saccharose, Laktose, Fructose, Maltose, Melasse, gegebenenfalls Stärke, gegebenenfalls Cellulose oder aus Glycerin und Ethanol, gegebenenfalls auch aus Mischungen, herstellen. Es handelt sich um Vertreter der Familie Enterobacteriaceae, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

Rekombinante Mikroorganismen werden im Allgemeinen durch Transformation, Transduktion oder Konjugation, oder einer Kombination dieser Methoden, mit einem Vektor erzeugt, der das gewünschte Gen, den gewünschten ORF, ein Allel dieses Gens oder ORFs oder Teile davon und/oder einen die Exprimierung des Gens oder ORFs verstärkenden Promotor enthält. Bei diesem Promotor kann es sich um den durch verstärkende Mutation aus der eigenen, upstream des Gens oder ORFs befindlichen regulatorischen Sequenz hervorgegangenen Promotor handeln, oder es wurde ein effizienter Promotor mit dem Gen oder ORF fusioniert.

Zur Herstellung der L-Aminosäure-anreichernden Stämme der Familie Enterobacteriaceae, welche ein verstärktes uspA-Gen enthalten, werden bevorzugt Stämme verwendet (Ausgangsstämme bzw. Elternstämme), die bereits die Fähigkeit besitzen, die gewünschte L-Aminosäure in der Zelle anzureichern und/oder in das sie umgebende Nährmedium auszuscheiden bzw. in der Fermentationsbrühe zu akkumulieren. Hierfür wird in diesem Zusammenhang auch der Ausdruck "produzieren" verwendet. Insbesondere besitzen die für die Maßnahmen der Erfindung eingesetzten Stämme die Fähigkeit ≥ (mindestens) 0,25 g/l, ≥ 0,5 g/l, ≥ 1,0 g/l, ≥ 1,5 g/l, ≥ 2,0 g/l, ≥ 4 g/l oder ≥ 10 g/l an L-Aminosäure in ≤ (maximal) 120 Stunden, ≤ 96 Stunden, ≤ 48 Stunden, ≤ 36 Stunden, ≤ 24 Stunden oder ≤ 12 Stunden in der Zelle und/oder im Nährmedium bzw. der Fermentationsbrühe anzureichern bzw. zu akkumulieren. Hierbei kann es sich um Stämme handeln, die durch Mutagenese und Selektion, durch rekombinante DNA-Techniken oder durch eine Kombination beider Methoden hergestellt wurden.

Die genannten L-Aminosäuren-anreichernden Stämme produzieren eine oder mehrere, bevorzugt eine oder im Wesentlichen eine Aminosäure ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin, bevorzugt ausgewählt aus der Gruppe L-Threonin, L-Tryptophan, L-Lysin, L-Valin, L-Isoleucin und L-Homoserin. Der Begriff L-Aminosäure oder Aminosäure umfasst auch deren Salze.

Der Begriff " eine oder im Wesentlichen eine Aminosäure" berücksichtigt, dass zusätzlich zu der gewünschten L-Aminosäure eine oder mehrer weitere der genannten L-Aminosäuren (Nebenaminosäuren) produziert werden können. Der Anteil dieser Nebenaminosäuren beträgt ≥ 0 bis maximal 40%, bevorzugt ≥ 0 bis maximal 20%, besonders bevorzugt ≥ 0 bis maximal 10% und ganz besonders bevorzugt ≥ 0 bis maximal 5% bezogen auf die Menge der gewünschten L-Aminosäure.

Als zum Elternstamm geeignete, insbesondere L-Threonin produzierende bzw. ausscheidende Stämme der Gattung Escherichia, insbesondere der Art Escherichia coli sind beispielsweise zu nennen:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli TH 14.97 (WO 02/26993)
- Escherichia coli TH 21.97 (WO 02/26993)
- Escherichia coli BKIIM B-3996 (US-A-5,175,107)
- Escherichia coli BKIIM B-3996ΔtdhΔpckA/pVIC40 (WO 02/29080)
- Escherichia coli kat 13 (WO 98/04715)
- Escherichia coli Kat 69.9 (WO 02/26993)
- Escherichia coli KCCM-10132 (WO 00/09660)
- Escherichia coli KCCM-10168 (WO 01/14525)
- Escherichia coli KCCM-10133 (WO 00/09661)

Als zum Elternstamm geeignete L-Threonin produzierende bzw. ausscheidende Stämme der Gattung Serratia, insbesondere der Art Serratia marcescens sind beispielsweise zu nennen:
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (AppliedBiochemistry and Biotechnology 37(3): 255-265 (1992))

L-Threonin produzierende oder ausscheidende Stämme aus der Familie der Enterobacteriaceae besitzen bevorzugt, unter anderem, ein oder mehrere der genetischen bzw. phänotypischen Merkmale ausgewählt aus der Gruppe: Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Als zum Elternstamm geeignete, L-Tryptophan produzierende bzw. ausscheidende Stämme der Gattung Escherichia, insbesondere der Art Escherichia coli sind beispielsweise zu nennen:
- Escherichia coli JP4735/pMU3028 (US5,756,345)
- Escherichia coli JP6015/pMU91 (US5,756,345)
- Escherichia coli SV164(pGH5) (WO94/08031)
- E. coli AGX17(pGX44) (NRRL B-12263) (US4,371,614)
- E. coli AGX6(pGX50)aroP (NRRL B-12264) (US4,371,614)
- Escherichia coli AGX17/pGX50,pACKG4-pps (WO97/08333)
- Escherichia coli ATCC 31743 (CA1182409)
- E. coli C534/pD2310,pDM136 (ATCC 39795) (WO87/01130
- Escherichia coli JB102/p5LRPS2 (US5,939,295).

L-Tryptophan produzierende oder ausscheidende Stämme aus der Familie der Enterobacteriaceae besitzen bevorzugt, unter anderem, ein oder mehrere der genetischen bzw. phänotypischen Merkmale ausgewählt aus der Gruppe: Resistenz gegen 5-Methyl-DL-Tryptophan, Resistenz gegen 5-Fluoro-Tryptophan, Resistenz gegen 4-Methyl-DL-Tryptophan, Resistenz gegen 6-Methyl-DL-Tryptophan, Resistenz gegen 4-Fluoro-Tryptophan, Resistenz gegen 6-Fluoro-Tryptophan, Resistenz gegen Anthranilat, Resistenz gegen Tryptazan, Resistenz gegen Indol, Resistenz gegen Indol-Acrylsäure, Bedürftigkeit für Phenylalanin, Bedürftigkeit für Tyrosin, gegebenenfalls Fähigkeit zur Saccharose-Verwertung, Verstärkung des Tryptophan-Operons, bevorzugt der Anthranilat-Synthase bevorzugt der feed back resistenten Form, eine teilweise defekte Tryptophanyl-tRNA-Synthase, eine abgeschwächte Tryptophan-Aufnahme, eine defekte Tryptophanase, inaktivierte Repressorproteine, Verstärkung der Serin-Biosynthese, Verstärkung der Phophoenolpyruvat-Synthese, Verstärkung der D-Erythrose-4-Phosphat-Synthese, Verstärkung der 3-deoxy-D-arabino-heptulosonat-7-Phosphat(DHAP)-Synthese, Verstärkung der Chorismat-Biosynthese.

Als ein als Elternstamm geeigneter, L-Homoserin ausscheidender bzw. produzierender Stamm der Gattung Escherichia, insbesondere der Art Escherichia coli ist beispielsweise zu nennen:
Escherichia coli NZ10rhtA23/pAL4 (US 6,960,455).

L-Homoserin produzierende oder ausscheidende Stämme aus der Familie der Enterobacteriaceae besitzen bevorzugt, unter anderem, ein oder mehrere der genetischen bzw. phänotypischen Merkmale ausgewählt aus der Gruppe: Bedürftigkeit für L-Threonin, Bedürftigkeit für L-Methionin, Bedürftigkeit für L-Isoleucin, eine defekte Homoserin-Kinase, gegebenenfalls Fähigkeit zur Saccharose-Verwertung, Verstärkung der Homoserin-Dehydrogenase I/Aspartatkinase I, bevorzugt der feed back resistenten Form, Verstärkung des RhtA-Genproduktes.

Als zum Elternstamm geeignete, L-Lysin ausscheidende bzw. produzierende Stämme der Gattung Escherichia, insbesondere der Art Escherichia coli sind beispielsweise zu nennen:
- Escherichia coli pDA1/TOC21R (= CNCM 1-167) (FR-A-2511032)
- Escherichia coli NRRL B-12199 (US4,346,170)
- Escherichia coli NRRL B-12185 (US4,346,170).

Als zum Elternstamm geeignete, L-Isoleucin ausscheidende bzw. produzierende Stämme der Gattung Escherichia, insbesondere der Art Escherichia coli sind beispielsweise zu nennen:
- Escherichia coli H-8670 (FERM BP-4051) (US5, 460, 958)
- Escherichia coli H-8683 (FERM BP-4052) (US5, 460, 958)
- Escherichia coli FERM BP-3628 (US5, 362, 637)
- Escherichia coli FERM BP-3629 (US5, 362, 637)
- Escherichia coli FERM BP-3630 (US5, 362, 637)
- Escherichia coli H-9146 (FERM BP-5055) (US5, 695, 972)
- Escherichia coli H-9156 (FERM BP-5056) (US5,695,972).

L-Isoleucin produzierende oder ausscheidende Stämme aus der Familie der Enterobacteriaceae besitzen bevorzugt, unter anderem, ein oder mehrere der genetischen bzw. phänotypischen Merkmale ausgewählt aus der Gruppe: Resistenz gegen Isoleucin-Analoga wie Thiaisoleucin, Resistenz gegen Ethionin, Resistenz gegen Arginin-Hydroxamat, Resistenz gegen S-(2-Aminoethyl)-L-Cystein, Resistenz gegen D-Serin.

Als Elternstamm geeigneter, L-Valin ausscheidender bzw. produzierender Stamm der Gattung Escherichia, insbesondere der Art Escherichia coli ist beispielsweise zu nennen:
- Escherichia coli AJ11502 (NRRL B-12288) (US-A-4391907).

Bei den der Erfindung zugrunde liegenden Arbeiten wurde gefunden, dass Mikroorganismen der Familie Enterobacteriaceae nach Überexpression des Gens uspA, oder dessen Allelen, vermehrt L-Aminosäuren, insbesondere L-Threonin, L-Tryptophan, L-Lysin, L-Valin, L-Isoleucin und L-Homoserin, produzieren und in der Zelle oder im Medium anreichern.

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminopeptidase) die N-terminale Aminosäure Methionin abgespalten werden kann.

Aus den ebenfalls zur Familie Enterobacteriaceae gehörenden Salmonella enterica und Photorhabdus luminescens ist die Nukleotidsequenz für das uspA-Gen ebenso bekannt (Accession No.: AE014613 (REGION: 4063524-4063958 komplementär) und Accession No.: BX571859 (REGION: 126961-127392)). Weitere Nukleotidsequenzen für das uspA-Gen findet man aus folgenden Enterobacteriaceae: Shigella flexneri (Accession No.: CP000266, AE005674, AE014073); Shigella boydii (Accession No.: CP000036); Shigella dysenteriae (Accession No.: CP000034); Shigella sonnei (Accession No.: CP000038); Salmonella typhimurium (Accession No.: AE008865); Enterobacter (Accession No.: CP000653); Klebsiella pneumoniae (Accession No.: CP000647); Sodalis glossinidius (Accession No.: AP008232); Erwinia carotovora (Accession No.: BX950851).

Das uspA-Gen von Escherichia coli K12 wird unter anderem durch folgende Angaben beschrieben:

| | |
|---|---|
| Bezeichnung: | universelles Stressprotein A |
| Funktion: | Zell-Adaption und Zellschutz als Bestandteil einer SOS-Antwort |
| Referenz: | Nystrom T. und Neidhardt FC.; Molecular microbiology 6(21): 3187-3198 (1992) Diez et al.; Molecular microbiology 36(6): 1494-1503 (2000) |
| | Nachin et al.; Journal of Bacteriology 187 (18) : 6265-6272 (2005) |
| Accession No.: | NC_000913 (REGION: 3638134-3638568) Das kodierte Polypeptid besitzt eine Länge von 144 Aminosäuren. |
| | |
| Alternativer Genname: | ECK3480 , b3495 |

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannte Sequenz zum uspA-Gen von Escherichia coli unter der SEQ ID No. 1 und die bekannten Sequenzen zum uspA-Gen von Salmonella enterica und Photorhabdus luminescens unter den SEQ ID No. 3 und SEQ ID No. 5 dargestellt. Die von diesen Leserahmen kodierten Proteine sind als SEQ ID No. 2, SEQ ID No. 4 und SEQ ID No. 6 dargestellt.

Die in den angegebenen Textstellen beschriebenen Gene oder offenen Leserahmen können erfindungsgemäß verwendet werden. Weiterhin können Allele der Gene oder offene Leserahmen verwendet werden, die sich aus der Degeneriertheit des genetischen Codes oder durch funktionsneutrale Sinnmutationen ("sense mutations") ergeben. Die Verwendung endogener Gene bzw. endogener offener Leserahmen wird bevorzugt.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Zu den Allelen des uspA-Gens, welche funktionsneutrale Sinnmutationen enthalten, zählen unter anderem solche, die zu höchstens 15, bevorzugt zu höchstens 10 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 20, 10, 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 5, 8 in keinem Falle aber mehr als 10 Aminosäuren betreffen.

Zu den geeigneten Allelen gehören ferner solche, die durch Hybridisierung, insbesondere unter stringenten Bedingungen unter Verwendung von SEQ ID No. 1, SEQ ID No. 3 oder SEQ ID No. 5 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 75% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 75% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C-68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 75% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1, SEQ ID No. 3 oder SEQ ID No. 5 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Zur Erzielung einer Überexpression kann die Promotorregion, die sich stromaufwärts des Strukturgens, bevorzugt des uspA-Gens, befindet, mutiert werden. In gleicher Weise wirken Expressionskassetten oder Promotoren, die stromaufwärts des Strukturgens eingebaut werden. Durch induzierbare Promotoren ist es zusätzlich möglich, die Expression im Verlaufe der fermentativen L-Aminosäure-Produktion zu steigern. Des weiteren vorteilhaft sein kann auch die Verwendung von Promotoren zur Genexpression, die eine andere zeitliche Genexpression ermöglicht. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene, ORFs oder Genkonstrukte können entweder in Plasmiden mit unterschiedlicher Kopienzahl vorliegen oder im Chromosom integriert und amplifiziert sein.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die att-site des Phagen λ (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, lpp-Promotor oder P_{L}-Promotor und P_{R}-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren, der nar-Promotor oder die Promotoren der Gene rrsG, rnpB, csrA, csrB, ompA, fusA, pepQ, rplX oder rpsG verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression plasmidgebundener Gene kontrollieren (Glascock und Weickert, Gene 223, 221-231 (1998)), die Verwendung dieser Plasmide ohne Induktion durch IPTG-Zugabe (= Isopropyl-β-D-Thiogalactopyranosid) führt zu einer sehr schwachen Expression von ohne eigenem Promotor klonierten Genen. Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Der Austausch des Promotors gegen einen zum Beispiel in genomweit-vergleichenden Expressionsanalysen ermittelten Promotor mit gleichmäßig hoher Expression im Verlauf eines Fermentationsprozesses bewirkt eine gleichmäßige Verstärkung. Die Veränderung einer Wachstumsphasen-abhängigen Genexpression kann beispielsweise wie bei Walker et al. (Journal of Bacteriology 181: 1269-80 (1999)) beschrieben durch die Verwendung des Wachstumsphasen-abhängigen fis Promotors oder einer der dort beschriebenen Mutationen des fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden. Desweiteren kann der Austausch eines Start-Codons zu dem in Escherichia coli mit 77% am häufigsten vorkommenden Codon ATG die Translation erheblich verbessern, da das Codon AUG zwei- bis dreimal effektiver ist als zum Beispiel die Codons GUG und UUG (Khudyakov et al., FEBS Letters 232(2):369-71 (1988); Reddy et al., Proceedings of the National Academy of Sciences of the USA 82(17):5656-60 (1985)). Auch die Sequenzumgebung des Start-Codons kann optimiert werden, da zusammenwirkende Effekte zwischen dem Start-Codon und den flankierenden Bereichen beschrieben sind (Stenstrom et al., Gene 273(2):259-65 (2001); Hui et al., EMBO Journal 3(3):623-9 (1984)).

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartolome et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens das uspA-Gen, oder für dessen Genprodukt kodierende Nukleotidsequenzen oder Allele trägt.

Unter dem Begriff Transformation versteht man die Aufnahme einer isolierten Nukleinsäure durch einen Wirt (Mikroorganismus).

Zur Erzeugung von uspA-Allelen, die in den für das erfindungsgemäße Verfahren eingesetzten Mikroorganismen zum Einsatz kommen, können unter anderem im Stand der Technik beschriebene Methoden zur gerichteten Mutagenese verwendet werden.

Es können Verfahren der ortsgerichteten Mutagenese unter Verwendung mutagener Oligonukleotide (T. A. Brown: Gentechnologie für Einsteiger, Spektrum Akademischer Verlag, Heidelberg, 1993) oder der Polymerase-Kettenreaktion (PCR), wie sie im Handbuch von Gait: Oligonukleotide synthesis: A Practical Approach (IRL Press, Oxford, UK, 1984) oder von Newton und Graham (PCR, Spektrum Akademischer Verlag, Heidelberg, 1994) beschrieben sind, verwendet werden. Die erzeugten Mutationen können durch DNA-Sequenzierung beispielsweise nach der Methode von Sanger et al. (Proceedings of the National Academy of Science USA 74 (12): 5463-5467 (1977)) bestimmt und überprüft werden.

Zur Konstruktion von Punktmutationen im uspA-Gen kann zum Beispiel das Quick Change Site-Directed Mutagenesis Kit der Firma Stratagene (Amsterdam, Niederlande) verwendet werden. Bei Verwendung dieser Methoden wird das im Stand der Technik beschriebene uspA-Gen ausgehend von isolierter Gesamt-DNA eines Wildtypstammes mit Hilfe der Polymerase-Kettenreaktion (PCR) amplifiziert, in geeignete Plasmidvektoren kloniert, und die DNA anschließend dem Mutageneseverfahren unterworfen. Mittels "GeneSOEing" (Gene Splicing by Overlap Extension, Horton, Molecular Biotechnology 3: 93-98 (1995)) können die Punktmutationen schon per PCR erhalten werden.

Die erzeugten uspA-Allele können beispielsweise durch Transformation und das Verfahren des Gen- bzw. Allelaustausches in geeignete Stämme eingebaut werden.

Eine gebräuchliche Methode ist die von Hamilton et al. (Journal of Bacteriology 171, 4617 - 4622 (1989)) beschriebene Methode des Genaustauschs mit Hilfe eines konditional replizierenden pSC101-Derivates pMAK705 oder mit pKO3 (Link et al., Journal of Bacteriology 179 : 6228-6237 (1997)). Andere im Stand der Technik beschriebene Methoden wie beispielsweise die von Martinez-Morales et al. (Journal of Bacteriology 1999, 7143-7148 (1999)) oder die von Boyd et al. (Journal of Bacteriology 182, 842-847 (2000)) können gleichfalls benutzt werden.

Es ist ebenfalls möglich die erzeugten uspA-Allele durch Konjugation oder Transduktion in verschiedene Stämme zu überführen.

Die Mutationen von uspA können ebenfalls in Expressionsplasmiden durchgeführt werden, so dass die resultierenden Proteine überproduziert werden können.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Weiterhin kann es für die Produktion von L-Aminosäuren, insbesondere L-Threonin, mit Stämmen der Familie Enterobacteriaceae vorteilhaft sein, zusätzlich zur Verstärkung des uspA-Gens, ein oder mehrere Enzym(e) der bekannten Aminosäure-Biosynthesewege oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

Ebenso kann es für die Produktion von L-Trytophan mit Stämmen der Familie Enterobacteriaceae vorteilhaft sein, zusätzlich zur Verstärkung des uspA-Gens, ein oder mehrere Enzyme des bekannten Tryptophan-Biosyntheseweges oder Enzyme der Serin-Biosynthese oder Enzyme für die Produktion von 3-deoxy-D-arabino-heptulosonat-7-Phosphat (DHAP) und Chorismat zu verstärken. Die Verwendung endogener Gene wird im Allgemeinen bevorzugt.

So können beispielsweise für die Produktion von L-Threonin gleichzeitig eines oder mehrere der Gene, ausgewählt aus der Gruppe
- mindestens ein Gen des für die Aspartatkinase, die Homoserin-Dehydrogenase, die Homoserinkinase und die Threoninsynthase kodierenden thrABC-Operons (US-A-4,278,765),
- das für die Pyruvat-Carboxylase kodierende pyc-Gen von Corynebacterium glutamicum (WO 99/18228),
- das für die Phosphoenolpyruvat-Synthase kodierende pps-Gen (Molecular and General Genetics 231(2): 332-336 (1992); WO 97/08333),
- das für die Phosphoenolpyruvat-Carboxylase kodierende ppc-Gen (WO 02/064808),
- die für die Untereinheiten der Pyridin-Transhydrogenase kodierenden Gene pntA und pntB (European Journal of Biochemistry 158: 647-653 (1986); WO 95/11985),
- das Gen rhtC, welches für das Threoninresistenz vermittelnde Protein kodiert (EP-A-1 013 765),
- das für das Threonin-Export-Carrier-Protein kodierende thrE-Gen von Corynebacterium glutamicum (WO 01/92545),
- das für die Glutamat-Dehydrogenase kodierende gdhA-Gen (Nucleic Acids Research 11: 5257-5266 (1983); Gene 23: 199-209 (1983); DE19907347),
- das für die Phosphohistidin-Protein-Hexose-Phosphotransferase des Phosphotransferase-Systems PTS kodierende ptsH-Gen des ptsHIcrr-Operons (WO 03/004674),
- das für das Enzym I des Phosphotransferase-Systems PTS kodierende ptsI-Gen des ptsHIcrr-Operons (WO 03/004674),
- das für die Glucose-spezifische IIA Komponente des Phosphotransferase-Systems PTS kodierende crr-Gen des ptsHIcrr-Operons (WO 03/004674),
- das für die Glucose-spezifische IIBC Komponente kodierende ptsG-Gen (WO 03/004670),
- das für die Cystein-Synthase A kodierende cysK-Gen (WO 03/006666),
- das für den Regulator des cys-Regulons kodierende cysB-Gen (WO 03/006666),
- das für das Flavoprotein der NADPH-Sulfit-Reduktase kodierende cysJ-Gen des cysJIH-Operons (WO 03/006666),
- das für das Hämoprotein der NADPH-Sulfit-Reduktase kodierende cysI-Gen des cysJIH-Operons (WO 03/006666),
- das für die Adenylylsulfat-Reduktase kodierende cysH-Gen des cysJIH-Operons (WO 03/006666),
- das für die Decarboxylase Untereinheit der 2-Ketoglutarat Dehydrogenase kodierende sucA-Gen des sucABCD-Operons (WO 03/008614),
- das für die Dihydrolipoyltranssuccinase E2 Untereinheit der 2-Ketoglutarat Dehydrogenase kodierende sucB-Gen des sucABCD-Operons (WO 03/008614),
- das für die β-Untereinheit der Succinyl-CoA Synthetase kodierende sucC-Gen des suc ABCD-Operons (WO 03/008615),
- das für die α-Untereinheit der Succinyl-CoA Synthetase kodierende sucD-Gen des sucABCD-Operons (WO 03/008615),
- das Genprodukt des offenen Leserahmens (ORF) yjcG von Escherichia coli (Accession Number NC000913 (Region 4281276-4282925) des National Center for Biotechnology Information (NCBI, Bethesda, MD, USA),
- das Genprodukt des offenen Leserahmens (ORF) yibD von Escherichia coli (Accession Number AE000439 des National Center for Biotechnology Information (NCBI, Bethesda, MD, USA)),
- das Genprodukt des offenen Leserahmens (ORF) yaaU von Escherichia coli (Accession Number NC000913 (Region 45807-47138) des National Center for Biotechnology Information (NCBI, Bethesda, MD, USA), der auch unter der Bezeichnung yaaU-ORF bekannt ist,
- das für das universelle Stressprotein UspC kodierende uspC-Gen (Accession Number NC000913 (Region 1977777-1978205) des National Center for Biotechnology Information (NCBI, Bethesda, MD, USA)),
- das uspD-Gen, welches für das Resistenz gegen UV-Strahlung vermittelnde universelle Stressprotein UspD kodiert (Accession Number NC000913 (Region 4111316-4111744) des National Center for Biotechnology Information (NCBI, Bethesda, MD, USA)),
- das uspE-Gen, welches für das Resistenz gegen UV-Strahlung vermittelnde universelle Stressprotein UspE kodiert (Accession Number NC000913 (Region 1395696-1396646) des National Center for Biotechnology Information (NCBI, Bethesda, MD, USA)),
verstärkt, insbesondere.überexprimiert werden.

Desweiteren können beispielsweise für die Produktion von L-Tryptophan, gleichzeitig eines oder mehrere der Gene, ausgewählt aus der Gruppe
- mindestens ein Gen des für die Anthranilat-Synthase, die Antranilat-Phosphoribosyltransferase, die Phosphoribosylanthranilat-Isomerase, die Indol-3-Glyzerinphosphat-Synthase und die Tryptophan-Synthase kodierenden Tryptophan-Operons (Applied and Environmental Microbiology 38(2): 181-190 (1979)),
- das für die 3-Phosphoglycerat-Dehydrogenase kodierende serA-Gen (WO87/01130)
- das für die Phosphoserinphosphatase kodierende serB-Gen (WO87/01130)
- das für die Phosphoserinaminotransferase kodierende serC-Gen (WO87/01130)
- das für die L-Tyrosin sensitive DHAP-Synthase kodierende aroF-Gen (WO87/01130; EP1270721)
- das für die L-Phenylalanin sensitive DHAP-Synthase kodierende aroG-Gen (WO87/01130; EP1270721)
- das für die L-Tryptophan sensitive DHAP-Synthase kodierende aroH-Gen (WO87/01130; EP1270721)
- das für die Phosphoenolpyruvat-Synthase kodierende pps-Gen (WO96/08567)
- das für die Phosphoenolpyruvat-Carboxykinase kodierende pckA-Gen (WO2004/090125),
- das für die Transketolase A kodierende tktA-Gen (US5,168,056),
- das für die Transketolase B kodierende tktB-Gen (US5,168,056),
- das Genprodukt des offenen Leserahmens (ORF) yddG von Escherichia coli (Accession Number NC000913 (Region 1544312-1545193) des National Center for Biotechnology Information (NCBI, Bethesda, MD, USA)),
- das für das universelle Stressprotein UspC kodierende uspC-Gen (Accession Number NC000913 (Region 1977777-1978205) des National Center for Biotechnology Information (NCBI, Bethesda, MD, USA)),
- das für das Resistenz gegen UV-Strahlung vermittelnde universelle Stressprotein UspD kodierende uspD-Gen (Accession Number NC000913 (Region 4111316-4111744) des National Center for Biotechnology Information (NCBI, Bethesda, MD, USA)),
- das für das Resistenz gegen UV-Strahlung vermittelnde universelle Stressprotein UspE kodierende uspE-Gen (Accession Number NC000913 (Region 1395696-1396646) des National Center for Biotechnology Information (NCBI, Bethesda, MD, USA)),
verstärkt, insbesondere überexprimiert werden.

L-Threonin produzierende Mikroorganismen der Familie Enterobacteriaceae besitzen typischerweise eine "feed back" resistente bzw. desensibilisierte Aspartatkinase I/Homoserin-Dehydrogenase I. Unter "feed back" resistenter Aspartatkinase/Homoserin-Dehydrogenase versteht man Aspartatkinase/Homoserin-Dehydrogenase Enzyme (kodiert durch thrA, EC:2.7.2.4/ EC:1.1.1.3), die im Vergleich zur Wildform eine geringere Empfindlichkeit gegenüber der Hemmung durch Threonin oder Mischungen von Threonin und dem Threonin-Analogon α-Amino-β-Hydroxyvaleriansäure (AHV) oder AHV allein aufweisen. Die für diese desensibilisierten Enzyme kodierenden Gene bzw. Allele werden auch als thrA^{FBR}-Allele bezeichnet. Im Stand der Technik sind thrA^{FBR}-Allele beschrieben, die für Aspartatkinase/Homoserin-Dehydrogenase Varianten kodieren, welche Aminosäureaustausche im Vergleich zum Wildtypprotein besitzen. Die Kodierregion des Wildtyp thrA-Gens von Escherichia coli entsprechend der Zugangsnummer U00096.2 der NCBI Datenbank (Bethesda, MD, USA) ist in SEQ ID NO:7 und das von diesem Gen kodierte Polypeptid in SEQ ID NO:8 dargestellt.

Auch die Nukleotidsequenz des thrA-Gens von Serratia marcescens ist bekannt und unter der Zugangsnummer X60821 beim NCBI verfügbar. Die Kodierregion des Wildtyp thrA-Gens von Serratia marcescens ist in SEQ ID NO:9 und das von diesem Gen kodierte Polypeptid in SEQ ID NO:10 dargestellt.

Die für die Maßnahmen der Erfindung eingesetzten L-Threonin produzierenden Mikroorganismen der Familie Enterobacteriaceae verfügen bevorzugt über ein thrA-Allel, das für eine Aspartatkinase/Homoserin-Dehydrogenase Variante kodiert, welche die Aminosäuresequenz von SEQ ID NO:8 oder SEQ ID NO:10 besitzt, wobei diese eine oder mehrere der Aminosäureaustausche ausgewählt aus der Gruppe:
ThrA E253K (Austausch von L-Glutaminsäure an Position 253 des kodierten Enzyms Aspartatkinase/Homoserin-Dehydrogenase gemäß SEQ ID NO:8 oder SEQ ID NO:10 gegen L-Lysin; siehe Research Disclosure 505, 537 (2006)),
ThrA G330D (Austausch von Glycin an Position 330 des kodierten Enzyms Aspartatkinase/Homoserin-Dehydrogenase gemäß SEQ ID NO:8 oder SEQ ID NO:10 gegen L-Asparaginsäure; siehe Omori et al. (Journal of Bacteriology 175(3), 785-794 (1993)),
ThrA S345F (Austausch von L-Serin an Position 345 des kodierten Enzyms Aspartatkinase/Homoserin-Dehydrogenase gemäß SEQ ID NO:8 oder SEQ ID NO:10 gegen L-Phenylalanin; siehe Lee et al., Journal of Bacteriology 185(18): 5442-5451 (2003)),
ThrA S352, Austausch von L-Serin an Position 352 des kodierten Enzyms Aspartatkinase/Homoserin-Dehydrogenase gemäß SEQ ID NO:8 oder SEQ ID NO:10 gegen L-Phenylalanin, L-Tyrosin, L-Asparagin, L-Alanin, L-Arginin, L-Glutamin, L-Glutaminsäure, L-Histidin, L-Leucin, L-Methionin, L-Tryptophan oder L-Valin, bevorzugt gegen L-Phenylalanin; siehe Omori et al. (Journal of Bacteriology 175(3), 785-794 (1993) und Omori et al. (Journal of Bacteriology 175(4), 959-965 (1993),
ThrA A479T (Austausch von L-Alanin an Position 479 des kodierten Enzyms Aspartatkinase/Homoserin-Dehydrogenase gemäß SEQ ID NO:8 oder SEQ ID NO:10 gegen L-Threonin; siehe Omori et al. (Journal of Bacteriology 175(3), 785-794 (1993)),
umfasst.

Bevorzugt wird eines der thrA^{FBR}-Allele thrA E253K (Austausch von L-Glutaminsäure an Position 253 des kodierten Enzyms Aspartatkinase/Homoserin-Dehydrogenase gegen L-Lysin)oder S345F (Austausch von L-Serin an Position 345 des kodierten Enzyms Aspartatkinase/Homoserin-Dehydrogenase gegen L-Phenylalanin) gemäß SEQ ID NO:8.

Die hier beschriebenen thrA^{FBR}-Allele, die für ein Aspartatkinase/Homoserin-Dehydrogenase Enzym kodieren, können mit den oben beschriebenen Maßnahmen überexprimiert werden.

Gegebenenfalls kann es von Vorteil sein, nur eine gemäßigte Erhöhung der intrazellulären Aktivität oder Konzentration eines oder mehrerer Enzyme oder Proteine in einem Mikroorganismus durchzuführen, da eine zu starke Erhöhung zum Beispiel zu einer fehlerhaften Zellteilung oder veränderter Zellmorphologie bis hin zur Toxizität führen kann (Guthrie und Wickner, Journal of Bacteriology 172(10):5555-5562 (1990); Genevaux P. et al.; EMBO Reports 5(2): 195-200 (2004)).

Der Begriff "gemäßigte Erhöhung" beschreibt die Erhöhung der intrazellulären Aktivität oder Konzentration des entsprechenden Proteins um höchstens das 10-fache, das 8-fache, das 6-fache, das 4-fache, das 3-fache, das 2-fache oder das 1,5-fache bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm. Als nicht rekombinanter Mikroorganismus oder Elternstamm (parent strain) wird der Mikroorganismus verstanden, an dem die erfindungsgemäße Verstärkung oder Überexpression durchgeführt wird.

Weiterhin kann es für die Produktion von L-Aminosäuren, insbesondere L-Threonin, L-Tryptophan, L-Lysin, L-Valin, L-Isoleucin und L-Homoserin vorteilhaft sein, zusätzlich zur Verstärkung des uspA-Gens, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

So können beispielsweise für die Produktion von L-Threonin gleichzeitig eines oder mehrere der Gene, ausgewählt aus der Gruppe
- das für die Threonin-Dehydrogenase kodierende tdh-Gen (Journal of Bacteriology 169: 4716-4721 (1987)),
- das für die Malat-Dehydrogenase (E.C. 1.1.1.37) kodierende mdh-Gen (Archives in Microbiology 149: 36-42 (1987)),
- das für das Enzym Phosphoenolpyruvat-Carboxykinase kodierende pckA-Gen (WO 02/29080),
- das für die Pyruvat-Oxidase kodierende poxB-Gen (WO 02/36797),
- das für den DgsA-Regulator des Phosphotransferase-Systems kodierende dgsA-Gen (WO 02/081721), das auch unter der Bezeichnung mlc-Gen bekannt ist,
- das für den Fructose-Repressor kodierende fruR-Gen (WO 02/081698), das auch unter der Bezeichnung cra-Gen bekannt ist,
- das für den Sigma³⁸-Faktor kodierende rpoS-Gen (WO 01/05939), das auch unter der Bezeichnung katF-Gen bekannt ist,
- das für die Aspartat Ammonium-Lyase kodierende aspA-Gen (WO 03/008603),
abgeschwächt, insbesondere ausgeschaltet oder die Expression verringert werden. Diese Massnahmen werden gegebenenfalls zusätzlich zu bzw. in geeigneter Kombination mit den angegebenen Massnahmen der Verstärkung von Genen zur Erhöhung der Threonin-Produktion durchgeführt.

Weiterhin kann es für die Produktion von L-Tryptophan vorteilhaft sein, zusätzlich zur Verstärkung des uspA-Gens, eines oder mehrere der Gene ausgewählt aus der Gruppe
- das für die Tryptophanase kodierende tnaA-Gen (US4,371,614),
- das für den Repressor des trp-Operons kodierende trpR-Gen (US4,371,614)
- das für die Chorismat-Mutase T und Prephenat-Dehydrogenase kodierende tyrA-Gen (US4,371,614),
- das für die Chorismat-Mutase P und Prephenat-Dehydrogenase kodierende pheA-Gen (US4,371,614),
- das für das Tryptophan spezifische Transportprotein kodierende mtr-Gen (US5,756,345),
- das für die Tryptophan-Permease kodierende tnaB-Gen (US5, 756, 345),
- das für den Transporter für aromatische Aminosäuren kodierende aroP-Gen (US5,756,345),
- das für die L-Serin Deaminase kodierende sdaA-Gen (EP0149539),
- das für die Glukose-6-Phosphat-Isomerase kodierende pgi-Gen (WO87/01130),
- das für die Tyrosin-Aminotransferase kodierende tyrB-Gen (WO87/01130),
abzuschwächen, insbesondere auszuschalten oder die Expression zu verringern. Diese Massnahmen werden gegebenenfalls zusätzlich zu bzw. in geeigneter Kombination mit den angegebenen Massnahmen der Verstärkung von Genen zur Erhöhung der Tryptophan-Produktion durchgeführt.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im für das entsprechende Enzym bzw. Protein nicht rekombinanten Mikroorganismnus oder Elternstamm oder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfindungsgemäße Abschwächung oder Ausschaltung durchgeführt wird.

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)), Kawano et al. (Nucleic Acids Research 33(19), 6268-6276 (2005)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservative Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation.

Deletionen von mindestens einem (1) oder mehreren Kodonen führen typischerweise ebenfalls zu einem vollständigen Ausfall der Enzymaktivität. Die Verringerung der Genexpression durch Suppression einer Stop-Kodon-Mutation im Kodierbereich durch geeignete t-RNA-Suppressoren ist in der WO 03/074719 beschrieben.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182: 842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Gegebenenfalls können die Maßnahmen zur Verstärkung und zur Abschwächung beliebig kombiniert werden.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den nicht rekombinanten Mikroorganismus oder Elternstamm bzw. den Fermentationsprozess unter Verwendung desselben erhöht.

Erfindungsgemäß werden die hergestellten Mikroorganismen im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US5,763,230) kultiviert. Zusammenfassungen über derartige Verfahren sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Bei einem batch-Verfahren werden, mit wenigen Ausnahmen, wie beispielsweise Sauerstoff und pH-Korrekturmittel, sämtliche Einsatzstoffe in Form eines Ansatzes vorgelegt und der Mikrorganismus in dem erhaltenen Medium kultiviert.

Bei einem fed batch-Verfahren wird der Mikrorganismus zunächst mittels eines batch-Verfahrens kultiviert (batch-Phase). Im Anschluss daran wird der Kultur ein für die Herstellung des Produktes wesentlicher Einsatzstoff, ggf. auch mehrere Einsatzstoffe, kontinuierlich oder diskontinuierlich hinzugefügt (Zulaufphase, feed-Phase). Im Falle der Herstellung von L-Aminosäuren handelt es sich hierbei um eine Kohlenstoffquelle.

Bei einem repeated fed batch-Verfahren handelt es sich um ein fed batch-Verfahren, bei dem nach Abschluss der Fermentation ein Teil der erhaltenen Fermentationsbrühe als Inokulum zum Start eines erneuten repeated fed batch-Verfahrens dient. Dieser Zyklus kann ggf. mehrfach wiederholt werden. Repeated fed batch-Verfahren sind beispielsweise in der WO 02/18543 und WO 05/014843 beschrieben.

Bei einem kontinuierlichen Verfahren werden im Anschluß an ein batch- oder fed batch-Verfahren ein oder mehrere ggf. sämtliche Einsatzstoffe zu der Kultur kontinuierlich hinzugefügt und gleichzeitig Fermentationsbrühe entnommen. Kontinuierliche Verfahren sind beispielsweise in den Patentschriften US 5,763,230, WO 05/014840, WO 05/014841 und WO 05/014842 beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium, Fermentationsmedium und Nährmedium bzw. Medium sind gegenseitig austauschbar.

Im Allgemeinen enthält ein Kulturmedium unter anderem eine oder mehrere Kohlenstoffquelle(n), Stickstoffquelle(n) und Phosphorquelle(n).

Als Kohlenstoffquelle können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einzigen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Durch die Tätigkeit der Mikroorganismen kommt es zu einer Anreicherung bzw. Akkumulation der L-Aminosäure in der Fermentations- bzw. Kulturbrühe. Die Kultur wird solange fortgesetzt, bis sich ein Maximum an der gewünschten L-Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Unter einer Fermentationsbrühe bzw. Kulturbrühe versteht man ein Fermentationsmedium, in dem ein Mikroorganismus für eine gewisse Zeit und bei einer gewissen Temperatur kultiviert wurde.

Bei Abschluss der Fermentation enthält die entstandene Fermentationsbrühe dementsprechend a) die infolge der Vermehrung der Zellen des Mikroorganismus entstandene Biomasse (Zellmasse) des Mikroorganismus, b) die im Laufe der Fermentation gebildete L-Aminosäure, c) die im Laufe der Fermentation gebildeten organischen Nebenprodukte und d) die durch die Fermentation nicht verbrauchten Bestandteile des/ der eingesetzten Fermentationsmediums/ Fermentationsmedien bzw. der Einsatzstoffe wie beispielsweise Vitamine wie Thiamin oder Salze wie Magnesiumsulfat.

Die hergestellte Kultur- bzw. Fermentationsbrühe kann anschließend gesammelt und die gewünschte L-Aminosäure bzw. das L-Aminosäure-haltige Produkt gewonnen oder isoliert werden.

In einer Verfahrensvariante wird die Fermentationsbrühe gegebenenfalls konzentriert und anschließend die L-Aminosäure gereinigt bzw. in reiner oder nahezu reiner Form isoliert. Typische Methoden zur Reinigung von L-Aminosäuren sind die Ionenaustauschchromatographie, die Kristallisation, Extraktionsverfahren und die Behandlung mit Aktivkohle. Hierdurch erhält man weitgehend reine L-Aminosäuren mit einem Gehalt von ≥ 90 Gew.-%, ≥ 95 Gew.-%, ≥ 96 Gew.-%, ≥ 97 Gew.-% ≥ 98 Gew.-% oder ≥ 99 Gew.-%.

Es ist ebenfalls möglich in einer anderen Verfahrensvariante aus der hergestellten Fermentationsbrühe ein Produkt herzustellen, indem man die in der Fermentationsbrühe enthaltene Biomasse des Bakteriums vollständig (100%) oder nahezu vollständig d.h. mehr als oder größer als (>) 90%, >95%, >97%, >99% entfernt und die übrigen Bestandteile der Fermentationsbrühe weitgehend d.h. zu 30% - 100%, 40% - 100%, 50% - 100%, 60% - 100%, 70% - 100%, 80% - 100%, oder 90% - 100%, bevorzugt größer gleich (≥) 50%, ≥60%, ≥70%, ≥80%, ≥90% oder ≥95% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder GranulierVerfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10 Gew.-%, kleiner als Gew.-5%, kleiner als Gew.-4% oder kleiner 3 Gew.-% beträgt.

Die Analyse von L-Aminosäuren zur Bestimmung der Konzentration zu einem oder mehreren Zeitpunkt(en) im Verlauf der Fermentation kann durch Anionenaustauschchromatographie mit anschließender Ninhydrin Derivatisierung erfolgen, so wie bei Spackman et al. (Analytical Chemistry 30: 1190-1206 (1958)) beschrieben, oder sie kann durch reversed phase HPLC erfolgen, so wie bei Lindroth et al. (Analytical Chemistry 51: 1167-1174 (1979)) beschrieben.

Das erfindungsgemäße Verfahren dient zur fermentativen Herstellung von L-Aminosäuren, wie beispielsweise L-Threonin, L-Isoleucin, L-Valin, L-Methionin, L-Homoserin, L-Tryptophan und L-Lysin, insbesondere L-Threonin, L-Tryptophan, L-Lysin, L-Valin, L-Isoleucin und L-Homoserin.

Die vorliegende Erfindung wird im Folgenden anhand von Ausführungsbeispielen näher erläutert.

Verwendete Minimal- (M9) und Vollmedien (LB) für Escherichia coli sind von J.H. Miller (A Short Course in Bacterial Genetics (1992), Cold Spring Harbor Laboratory Press) beschrieben. Die Isolierung von Plasmid-DNA aus Escherichia coli sowie alle Techniken zur Restriktion, Ligation, Klenow- und alkalische Phosphatasebehandlung werden nach Sambrook et al. (Molecular Cloning - A Laboratory Manual (1989) Cold Spring Harbor Laboratory Press) durchgeführt. Die Transformation von Escherichia coli wird, wenn nicht anders beschrieben, nach Chung et al. (Proceedings of the National Academy of Sciences of the United States of America 86: 2172-2175 (1989)) durchgeführt.

Die Bebrütungstemperatur bei der Herstellung von Stämmen und Transformanten ist 37°C.

### Beispiel 1

### Konstruktion des Expressionsplasmides pTrc99AuspA

Das uspA-Gen aus E. coli K12 wurde unter Anwendung der Polymerase-Kettenreaktion (PCR) sowie synthetischen Oligonukleotiden amplifiziert. Ausgehend von der Nukleotidsequenz des uspA-Gens in E. coli K12 MG1655 (Accession Number NC_000913 (Region: (3638134-3638568)), Blattner et al. (Science 277: 1453-1474 (1997)) wurden PCR-Primer synthetisiert (MWG Biotech, Ebersberg, Deutschland). Die Sequenzen der Primer wurden so modifiziert, dass Erkennungsstellen für Restriktionsenzyme entstehen. Für den uspA-1-Primer wurde die Erkennungssequenz für XbaI und für den uspA-2-Primer die Erkennungssequenz für HindIII gewählt, die in der unten dargestellten Nukleotidabfolge durch Unterstreichen markiert sind:
uspA-1:
   5' - TCTAGA GTGACGTAAC GGCACAAGAA- 3' (SEQ ID No. 11)
uspA-2:
   5' - AAGCTT TTCAGGAACA CGTCGTAGAG- 3' (SEQ ID No. 12)

Die für die PCR eingesetzte chromosomale E. coli K12 MG1655 DNA wurde nach Herstellerangaben mit "Qiagen Genomic-tips 100/G" (QIAGEN, Hilden, Deutschland) isoliert. Ein 539 bp großes DNA-Fragment konnte mit den spezifischen Primern unter Standard-PCR-Bedingungen (Innis et al. (1990) PCR Protocols. A Guide to Methods and Applications, Academic Press) mit der Vent-DNA-Polymerase (New England Biolaps GmbH, Frankfurt, Deutschland) amplifiziert werden (SEQ ID No. 13).

Das amplifizierte uspA-Fragment wurde mit dem Vektor pCR-Blunt II-TOPO (Zero TOPO TA Cloning Kit, Invitrogen, Groningen, Niederlande) den Herstellerangaben entsprechend ligiert und in den E. coli Stamm TOP10 transformiert. Die Selektion Plasmid tragender Zellen erfolgte auf LB Agar, der mit 50 µg/ml Kanamycin versetzt war. Nach der Plasmid DNA Isolierung wurde der Vektor mit dem Enzym EcoRI gespalten und nach Überprüfung der Spaltung im 0,8%igen Agarosegel mit pCRBluntuspA bezeichnet.

Die Nukleotidsequenz des amplifizierten DNA-Fragmentes bzw. PCR-Produktes wurde nach der Dideoxy-Kettenabbruch-Methode von Sanger et al. (Proceedings of the National Academy of Sciences U.S.A., 74:5463-5467 (1977)) mit dem "ABI Prism 377" Sequenziergerät der Firma PE Applied Biosystems (Weiterstadt, Deutschland) überprüft. Die Sequenz des PCR-Produktes entsprach den Positionen 1-435 der in der SEQ ID No. 1 dargestellten Sequenz. Die Aminosäuresequenz des dazugehörigen UspA-Proteins ist in der SEQ ID No. 14 dargestellt.

Anschließend wurde der Vektor pCRBluntuspA mit den Enzymen HindIII und XbaI gespalten und das ca. 533bp uspA-Fragment nach Auftrennung im 0,8%igem Agarosegel aus dem Gel isoliert (QIAquick Gel Extraction Kit, QIAGEN, Hilden, Deutschland) und mit dem Vektor pTrc99A (Pharmacia Biotech, Uppsala, Schweden), der mit den Enzymen HindIII und XbaI verdaut worden war, ligiert. Der E. coli Stamm DH5α (Grant et al.; Proceedings of the National Academy of Sciences USA, 87 (1990) 4645-4649) wurde mit dem Ligationsansatz transformiert und Plasmid tragende Zellen auf LB Agar, der mit 50 µg/ml Ampicillin versetzt ist, selektioniert.

Die erfolgreiche Klonierung konnte nach der Plasmid DNA Isolierung durch die Kontrollspaltung mit dem Enzym SalI nachgewiesen werden.

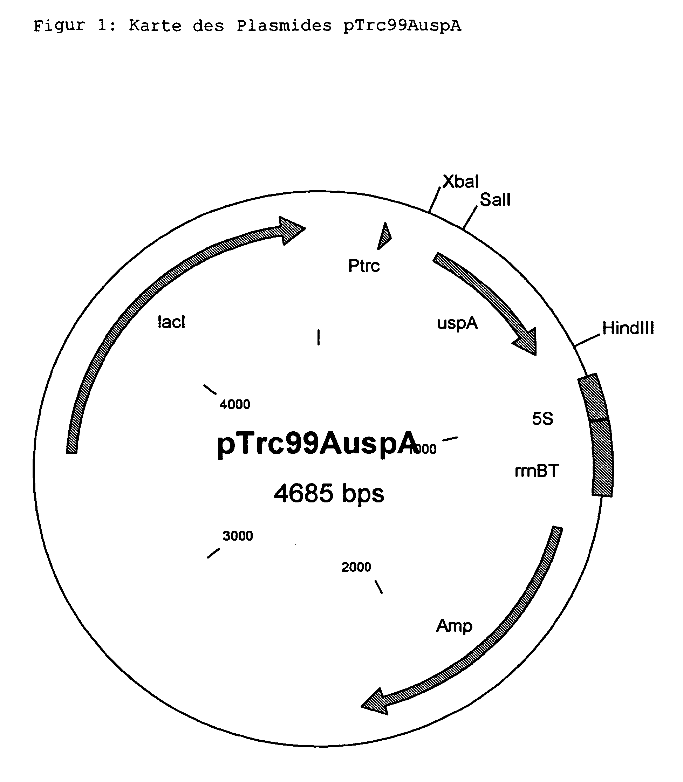

Das Plasmid wird als pTrc99AuspA (Figur 1) bezeichnet.

### Beispiel 2

### Herstellung von L-Threonin mit dem Stamm MG442/pTrc99AuspA

Der L-Threonin produzierende E. coli Stamm MG442 ist in der Patentschrift US-A- 4,278,765 beschrieben und bei der Russischen Nationalsammlung für industrielle Mikroorganismen (VKPM, Moskau, Russland) als CMIM B-1628 hinterlegt.

Der Stamm MG442 wurde mit dem in Beispiel 1 beschriebenen Expressionsplasmid pTrc99AuspA und mit dem Vektor pTrc99A transformiert und auf LB Agar mit 50 µg/ml Ampicillin Plasmid tragende Zellen selektioniert. Auf diese Weise entstanden die Stämme MG442/pTrc99AuspA und MG442/pTrc99A. Ausgewählte Einzelkolonien wurden anschließend auf Minimalmedium mit der folgenden Zusammensetzung: 3,5 g/l Na₂HPO₄*2H₂O, 1,5 g/l KH₂PO₄, 1 g/l NH₄Cl, 0,1 g/l MgSO₄*7H₂O, 2 g/l Glucose, 20 g/l Agar, 50 mg/l Ampicillin, weiter vermehrt. Die Bildung von L-Threonin wurde in batch Kulturen von 10 ml, die in 100 ml Erlenmeierkolben enthalten waren, überprüft. Dazu wurde 10 ml Vorkulturmedium der folgenden Zusammensetzung: 2 g/l Hefeextrakt, 10 g/l (NH₄)₂SO₄, 1 g/l KH₂PO₄, 0,5 g/l MgSO₄*7H₂O, 15 g/l CaCO₃, 20 g/l Glucose, 50 mg/l Ampicillin, beimpft und für 16 Stunden bei 37°C und 180 rpm auf einem ESR Inkubator der Firma Kühner AG (Birsfelden, Schweiz) inkubiert. Je 250 µl dieser Vorkultur wurden in 10 ml Produktionsmedium (25 g/l (NH₄)₂SO₄, 2 g/l KH₂PO₄, 1 g/l MgSO₄*7H₂O, 0,03 g/l FeSO₄*7H₂O, 0,018 g/l MnSO₄*1H₂O, 30 g/l CaCO₃, 20 g/l Glucose, 50 mg/l Ampicillin) überimpft und für 48 Stunden bei 37°C inkubiert. Nach der Inkubation wurde die optische Dichte (OD) der Kultursuspension mit einem LP2W-Photometer der Firma Dr. Lange (Düsseldorf, Deutschland) bei einer Messwellenlänge von 660 nm bestimmt.

Anschließend wurde die Konzentration an gebildetem L-Threonin im steril filtrierten Kulturüberstand mit einem Aminosäureanalysator der Firma Eppendorf-BioTronik (Hamburg, Deutschland) durch Ionenaustauschchromatographie und Nachsäulenreaktion mit Ninhydrindetektion bestimmt.

In Tabelle 1 ist das Ergebnis des Versuches dargestellt.

| Stamm | OD (660 nm) | L-Threonin g/l |
|---|---|---|
| MG442/pTrc99A | 3,0 | 1,3 |
| MG442/pTrc99AuspA | 5,9 | 1,9 |

### Kurze Beschreibung der Figuren:

- Figur 1:: Karte des das uspA-Gen enthaltenden Plasmides pTrc99AuspA

Längenangaben sind als ca.-Angaben aufzufassen. Die verwendeten Abkürzungen und Bezeichnungen haben folgende Bedeutung:
- Amp: Gen, welches für die Ampicillinresistenz kodiert
- lacI: Gen für das Repressorprotein des trc-Promotors
- Ptrc: trc-Promotorregion, IPTG-induzierbar
- uspA: Kodierregion des uspA-Gens
- 5S: 5S rRNA-Region
- rrnBT: rRNA-Terminator-Region

Die Abkürzungen für die Restriktionsenzyme haben folgende Bedeutung:
- XbaI: Restriktionsendonuklease aus Xanthomonas badrii
- HindIII: Restriktionsendonuklease aus Haemophilus influenzae
- SalI:Restriktionsendonuklease aus Streptomyces albus G

## Patentansprüche

1. Verfahren zur Herstellung von L-Aminosäuren durch Fermentation von rekombinanten Mikroorganismen der Familie Enterobacteriaceae, die ein überexprimiertes uspA-Gen enthalten, welches für das universelle Stressprotein A kodiert, **dadurch gekennzeichnet, dass** man
a) die die gewünschte L-Aminosäure produzierenden Mikroorganismen in einem Medium kultiviert unter Bedingungen, bei denen die gewünschte L-Aminosäure im Medium oder in den Zellen angereichert wird, und
b) die gewünschte L-Aminosäure isoliert, wobei Bestandteile der Fermentationsbrühe und/oder die Biomasse in ihrer Gesamtheit oder Anteilen (≥ 0 bis 100%) im isolierten Produkt verbleiben oder vollständig entfernt werden,wobei in den zur Herstellung von L-Aminosäuren fermentierten Mikroorganismen die Überexpression des uspA-Gens **dadurch** erzielt wird, dass man einen starken Promotor funktionell mit dem uspA-Gen verknüpft.

2. Verfahren gemäss Anspruch 1, **dadurch gekennzeichnet, dass** man zur Herstellung von L-Aminosäuren Mikroorganismen fermentiert, in denen ein dem uspA-Gen entsprechendes Polynukleotid überexprimiert wird, das für ein Polypeptid kodiert, dessen Aminosäuresequenz zu mindestens 80% identisch ist mit einer Aminosäuresequenz, ausgewählt aus der Gruppe SEQ ID No. 2, SEQ ID No. 4 und SEQ ID No. 6 .

3. Verfahren gemäss Anspruch 1, **dadurch gekennzeichnet, dass** man zur Herstellung von L-Aminosäuren Mikroorganismen fermentiert, welche ein dem uspA-Gen entsprechendes isoliertes Polynukleotid enthalten ausgewählt aus der Gruppe:
a) Polynukleotid mit einer Nukleotidsequenz, ausgewählt aus SEQ ID No. 1, SEQ ID No. 3, SEQ ID No. 5 und den dazu komplementären Nukleotidsequenzen;
b) Polynukleotid mit einer Nukleotidsequenz, die der SEQ ID No. 1, SEQ ID No. 3 oder SEQ ID No. 5 im Rahmen der Degeneration des genetischen Codes entspricht;
c) Polynukleotidsequenz mit einer Sequenz, die mit der zur Sequenz SEQ ID No. 1, SEQ ID No. 3 oder SEQ ID No. 5 komplementären Sequenz unter stringenten Bedingungen hybridisiert, wobei die stringenten Bedingungen durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1, SEQ ID No. 3 oder SEQ ID No. 5, die funktionsneutrale Sinnmutanten enthält,
wobei die Polynukleotide für das universelle Stressprotein A kodieren.

4. Verfahren gemäss Anspruch 2, **dadurch gekennzeichnet, dass** man zur Herstellung von L-Aminosäuren Mikroorganismen fermentiert, **dadurch gekennzeichnet, dass** das Polypeptid eine Aminosäuresequenz besitzt, die zu wenigstens 95% identisch ist mit einer der Sequenzen ausgewählt aus der Gruppe SEQ ID No. 2, SEQ ID No. 4 und SEQ ID No. 6.

5. Verfahren gemäss Anspruch 2, **dadurch gekennzeichnet, dass** man zur Herstellung von L-Aminosäuren Mikroorganismen fermentiert, **dadurch gekennzeichnet, dass** das Polypeptid die Aminosäuresequenz aufweist, die 100% identisch ist mit einer der Sequenzen SEQ ID No. 2 oder SEQ ID No. 4.

6. Verfahren gemäss den Anspruchen 1 bis 5, **dadurch gekennzeichnet, dass** als starker Promotor die Promotoren tac-Promotor, trp-Promotor, lpp-Promotor oder der P_{L}-Promotor oder P_{R}-Promotor des Phagen λ oder die Promotoren des Phagen T7, die gear-box-Promotoren, der nar-Promotor oder die Promotoren der Gene rrsG, rnpB, csrA, csrB, ompA, fusA, pepQ, rplX oder rpsG verwendet werden.

7. Verfahren gemäss den Ansprüchen 1 bis 6, **dadurch gekennzeichnet, dass** man zur Herstellung von L-Aminosäuren Mikroorganismen fermentiert, die durch Transformation, Transduktion, Konjugation oder eine Kombination dieser Methoden hergestellt werden, mit einem Vektor, der das uspA-Gen, ein Allel dieses Gens oder Teile davon, und einen Promotor enthält.

8. Verfahren gemäss den Ansprüchen 1 bis 7, **dadurch gekennzeichnet, dass** man zur Herstellung von L-Aminosäuren Mikroorganismen fermentiert, **dadurch gekennzeichnet, dass** die Mikroorganismen aus den Gattungen Escherichia, Erwinia, Providencia und Serratia ausgewählt sind.

9. Verfahren gemäss den Ansprüchen 1 bis 8, **dadurch gekennzeichnet, dass** man zur Herstellung von L-Aminosäuren Mikroorganismen fermentiert, **dadurch gekennzeichnet, dass** sie L-Threonin, L-Tryptophan, L-Lysin, L-Valin, L-Isoleucin, L-Methionin oder L-Homoserin produzieren.

10. Verfahren gemäss den Ansprüchen 1 bis 9, **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) mindestens ein Gen des für die Aspartatkinase, die Homoserin-Dehydrogenase, die Homoserinkinase und die Threoninsynthase kodierenden thrABC-Operons,
b) das für die Pyruvat-Carboxylase kodierende pyc-Gen von Corynebacterium glutamicum,
c) das für die Phosphoenolpyruvat-Synthase kodierende pps-Gen,
d) das für die Phosphoenolpyruvat-Carboxylase kodierende ppc-Gen,
e) die für die Untereinheiten der Pyridin-Transhydrogenase kodierenden Gene pntA und pntB,
f) das Gen rhtC, welches für das Threoninresistenz vermittelnde Protein kodiert,
g) das für das Threonin-Export-Carrier-Protein kodierende thrE-Gen aus Corynebacterium glutamicum,
h) das für die Glutamat-Dehydrogenase kodierende gdhA-Gen,
i) das für die Phosphohistidin-Protein-Hexose-Phosphotransferase kodierende ptsH-Gen,
j) das für das Enzym I des Phosphotransferase-Systems kodierende ptsI-Gen,
k) das für die Glucose-spezifische IIA Komponente kodierende crr-Gen,
l) das für die Glucose-spezifische IIBC Komponente kodierende ptsG-Gen,
m) das für die Cystein-Synthase A kodierende cysK-Gen,
n) das für den Regulator des cys-Regulons kodierende cysB-Gen,
o) das für das Flavoprotein der NADPH-Sulfit-Reduktase kodierende cysJ-Gen,
p) das für das Hämoprotein der NADPH-Sulfit-Reduktase kodierende cysI-Gen,
q) das für die Adenylylsulfat-Reduktase kodierende cysH-Gen,
r) das für die Decarboxylase Untereinheit der 2-Ketoglutarat Dehydrogenase kodierende sucA-Gen,
s) das für die Dihydrolipoyltranssuccinase E2 Untereinheit der 2-Ketoglutarat Dehydrogenase kodierende sucB-Gen,
t) das für die β-Untereinheit der Succinyl-CoA Synthetase kodierende sucC-Gen,
u) das für die α-Untereinheit der Succinyl-CoA Synthetase kodierende sucD-Gen,
v) das Genprodukt des offenen Leserahmens (ORF) yjcG von Escherichia coli,
w) das Genprodukt des offenen Leserahmens (ORF) yibD von Escherichia coli,
x) das Genprodukt des offenen Leserahmens (ORF) yaaU von Escherichia coli,
y) das für das universelle Stressprotein UspC kodierende uspC-Gen,
z) das uspD-Gen, welches für das Resistenz gegen UV-Strahlung vermittelnde universelle Stressprotein UspD kodiert und
aa) das uspE-Gen, welches für das Resistenz gegen UV-Strahlung vermittelnde universelle Stressprotein UspE kodiert,
überexprimiert.

11. Verfahren gemäss den Ansprüchen 1 bis 9, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
a) mindestens ein Gen des für die Anthranilat-Synthase, die Antranilat-Phosphoribosyltransferase, die Phosphoribosylanthranilat-Isomerase, die Indol-3-Glyzerinphosphat-Synthase und die Tryptophan-Synthase kodierenden Tryptophan-Operons,
b) das für die 3-Phosphoglycerat-Dehydrogenase kodierende serA-Gen,
c) das für die Phosphoserinphosphatase kodierende serB-Gen,
d) das für die Phosphoserinaminotransferase kodierende serC-Gen,
e) das für die L-Tyrosin sensitive DHAP-Synthase kodierende aroF-Gen,
f) das für die L-Phenylalanin sensitive DHAP-Synthase kodierende aroG-Gen,
g) das für die L-Tryptophan sensitive DHAP-Synthase kodierende aroH-Gen,
h) das für die Phosphoenolpyruvat-Synthase kodierende pps-Gen,
i) das für die Phosphoenolpyruvat-Carboxykinase kodierende pckA-Gen,
j) das für die Transketolase A kodierende tktA-Gen,
k) das für die Transketolase B kodierende tktB-Gen,
l) das Genprodukt des offenen Leserahmens (ORF) yddG von Escherichia coli,
m) das für das universelle Stressprotein UspC kodierende uspC-Gen,
n) das für das Resistenz gegen UV-Strahlung vermittelnde universelle Stressprotein UspD kodierende uspD-Gen und
o) das für das Resistenz gegen UV-Strahlung vermittelnde universelle Stressprotein UspE kodierende uspE-Gen,
überexprimiert.

12. Verfahren gemäss den Ansprüchen 1 bis 9, **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) das für die Threonin-Dehydrogenase kodierende tdh-Gen,
b) das für die Malat-Dehydrogenase kodierende mdh-Gen,
c) das für die Phosphoenolpyruvat-Carboxykinase kodierende pckA-Gen,
d) das für die Pyruvat-Oxidase kodierende poxB-Gen,
e) das für den DgsA-Regulator des Phosphotransferase-Systems kodierende dgsA-Gen,
f) das für den Fructose-Repressor kodierende fruR-Gen,
g) das für den Sigma³⁸-Faktor kodierende rpoS-Gen, und
h) das für die Aspartat Ammonium-Lyase kodierende aspA-Gen,
abschwächt, insbesondere ausschaltet oder die Expression verringert.

13. Verfahren gemäss den Ansprüchen 1 bis 9 oder 11, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
a) das für die Tryptophanase kodierende tnaA-Gen,
b) das für den Repressor des trp-Operons kodierende trpR-Gen,
c) das für die Chorismat-Mutase T und Prephenat-Dehydrogenase kodierende tyrA-Gen,
d) das für die Chorismat-Mutase P und Prephenat-Dehydrogenase kodierende pheA-Gen,
e) das für das Tryptophan spezifische Transportprotein kodierende mtr-Gen,
f) das für die Tryptophan-Permease kodierende tnaB-Gen,
g) das für den Transporter für aromatische Aminosäuren kodierende aroP-Gen,
h) das für die L-Serin Deaminase kodierende sdaA-Gen,
i) das für die Glukose-6-Phosphat-Isomerase kodierende pgi-Gen, und
j) das für die Tyrosin-Aminotransferase kodierende tyrB-Gen,
abschwächt, insbesondere ausschaltet oder die Expression verringert.

14. Verfahren gemäss den Ansprüchen 1 bis 9, **dadurch gekennzeichnet, dass** man L-Aminosäuren, ausgewählt aus der Gruppe L-Threonin, L-Tryptophan, L-Lysin, L-Valin, L-Isoleucin und L-Homoserin herstellt.

15. Verfahren gemäss den Ansprüchen 1 bis 14, **dadurch gekennzeichnet, dass** die Mikroorganismen in einem batch - Verfahren, einem fed batch - Verfahren, einem repeated fed batch-Verfahren oder in einem kontinuierlichen Verfahren kultiviert werden.

16. Verfahren gemäss den Ansprüchen 1 bis 15, **dadurch gekennzeichnet, dass** die Konzentration der L-Aminosäure zu einem oder mehreren Zeitpunkt (en) im Verlauf der Fermentation bestimmt wird.

## Claims

1. Process for the production of L-amino acids by fermentation of recombinant microorganisms of the family Enterobacteriaceae, which contain an overexpressed uspA gene which codes for the universal stress protein A, **characterized in that**
a) the microorganisms producing the desired L-amino acid are cultured in a medium under conditions in which the desired L-amino acid is enriched in the medium or in the cells, and
b) the desired L-amino acid is isolated, where constituents of the fermentation broth and/or the biomass remain in the isolated product in their entirety or in part (≥ 0 to 100%) or are completely removed, where overexpression of the uspA gene in the microorganisms fermented for the production of L-amino acids is achieved by functionally linking a strong promoter to the uspA gene.

2. Process according to Claim 1, **characterized in that** for the production of L-amino acids, microorganisms are fermented in which a polynucleotide corresponding to the uspA gene is overexpressed that codes for a polypeptide whose amino acid sequence is identical to at least 80% with an amino acid sequence selected from the group consisting of SEQ ID No. 2, SEQ ID No. 4 and SEQ ID No. 6.

3. Process according to Claim 1, **characterized in that** for the production of L-amino acids, microorganisms are fermented which contain an isolated polynucleotide corresponding to the uspA gene selected from the group consisting of:
a) polynucleotide having a nucleotide sequence selected from SEQ ID No. 1, SEQ ID No. 3, SEQ ID No. 5 and the nucleotide sequences complementary thereto;
b) polynucleotide having a nucleotide sequence that corresponds to the SEQ ID No. 1, SEQ ID No. 3 or SEQ ID No. 5 within the framework of the degeneration of the genetic code;
c) polynucleotide sequence having a sequence that hybridizes under stringent conditions with the sequence complementary to the sequence SEQ ID No. 1, SEQ ID No. 3 or SEQ ID No. 5, where the stringent conditions are achieved by a washing step in which the temperature extends over a range from 64°C to 68°C and the salt concentration of the buffer extends over a range from 2xSSC to 0.1xSSC;
d) polynucleotide having a sequence SEQ ID No. 1, SEQ ID No. 3 or SEQ ID No. 5, which contains functionally neutral sense mutants,
where the polynucleotides code for the universal stress protein A.

4. Process according to Claim 2, **characterized in that** for the production of L-amino acids, microorganisms are fermented, **characterized in that** the polypeptide has an amino acid sequence that is identical to at least 95% with one of the sequences selected from the group consisting of SEQ ID No. 2, SEQ ID No. 4 and SEQ ID No. 6.

5. Process according to Claim 2, **characterized in that** for the production of L-amino acids, microorganisms are fermented, **characterized in that** the polypeptide has the amino acid sequence that is 100% identical to one of the sequences SEQ ID No. 2 or SEQ ID No. 4.

6. Process according to Claims 1 to 5, **characterized in that** the promoters tac promoter, trp promoter, lpp promoter, or the λ phage P_{L} promoter or P_{R} promoter, or the T7 phage promoters, the gear-box promoters, the nar promoter, or the promoters of the genes rrsG, rnpB, csrA, csrB, ompA, fusA, pepQ, rplx or rpsG are used as strong promoter.

7. Process according to one of Claims 1 to 6, **characterized in that** for the production of L-amino acids, microorganisms that are produced by transformation, transduction, conjugation or a combination of these methods are fermented with a vector that contains the uspA gene, an allele of this gene or parts thereof and a promoter.

8. Process according to Claims 1 to 7, **characterized in that** for the production of L-amino acids, microorganisms are fermented, **characterized in that** the microorganisms are selected from the genera Escherichia, Erwinia, Providencia and Serratia.

9. Process according to Claims 1 to 8, **characterized in that** for the production of L-amino acids, microorganisms are fermented, **characterized in that** they produce L-threonine, L-tryptophan, L-lysine, L-valine, L-isoleucine, L-methionine or L-homoserine.

10. Process according to Claims 1 to 9, **characterized in that** for the production of L-threonine microorganisms are fermented, in which additionally at the same time one or more of the genes selected from the group consisting of:
a) at least one gene of the thrABC operon coding for aspartate kinase, homoserine dehydrogenase, homoserine kinase and threonine synthase,
b) the pyc gene of Corynebacterium glutamicum coding for pyruvate carboxylase,
c) the pps gene coding for phosphoenol pyruvate synthase,
d) the ppc gene coding for phosphoenol pyruvate carboxylase,
e) the genes pntA and pntB coding for the subunits of pyridine transhydrogenase,
f) the gene rhtC, which codes for the threonine resistance-mediating protein,
g) the thrE gene from Corynebacterium glutamicum coding for the threonine export carrier protein,
h) the gdhA gene coding for glutamate dehydrogenase,
i) the ptsH gene coding for the phosphohistidine protein hexose phosphotransferase,
j) the ptsI gene coding for the enzyme I of the phosphotransferase system,
k) the crr gene coding for the glucose-specific IIA component,
l) the ptsG gene coding for the glucose-specific IIBC component,
m) the cysK gene coding for cysteine synthase A,
n) the cysB gene coding for the regulator of the cys regulon,
o) the cysJ gene coding for the flavoprotein of NADPH sulphite reductase,
p) the cysI gene coding for the haemoprotein of NADPH sulphite reductase,
q) the cysH gene coding for adenylyl sulphate reductase,
r) the sucA gene coding for the decarboxylase subunit of 2-ketoglutarate dehydrogenase,
s) the sucB gene coding for the dihydrolipoyltranssuccinase E2 subunit of 2-ketoglutarate dehydrogenase,
t) the sucC gene coding for the β-subunit of succinyl-CoA synthetase,
u) the sucD gene coding for the α-subunit of succinyl-CoA synthetase,
v) the gene product of the open reading frame (ORF) yjcG of Escherichia coli,
w) the gene product of the open reading frame (ORF) yibD of Escherichia coli,
x) the gene product of the open reading frame (ORF) yaaU of Escherichia coli,
y) the uspC gene coding for the universal stress protein UspC,
z) the uspD gene, which codes for the resistance against UV radiation-mediating universal stress protein UspD and
aa)the uspE gene, which codes for the resistance against UV radiation-mediating universal stress protein UspE,
are overexpressed.

11. Process according to Claims 1 to 9, **characterized in that** for the production of L-tryptophan microorganisms of the family Enterobacteriaceae are fermented, in which additionally at the same time one or more of the genes selected from the group consisting of:
a) at least one gene of the tryptophan operon coding for anthranilate synthase, anthranilate phosphoribosyl transferase, phosphoribosyl anthranilate isomerase, indole-3-glycerol phosphate synthase and tryptophan synthase,
b) the serA gene coding for 3-phosphoglycerate dehydrogenase,
c) the serB gene coding for phosphoserine phosphatase,
d) the serC gene coding for phosphoserine aminotransferase,
e) the aroF gene coding for L-tyrosine-sensitive DHAP synthase,
f) the aroG gene coding for L-phenylalanine-sensitive DHAP synthase,
g) the aroH gene coding for L-tryptophan-sensitive DHAP synthase,
h) the pps gene coding for phosphoenol pyruvate synthase,
i) the pckA gene coding for phosphoenol pyruvate carboxykinase,
j) the tktA gene coding for transketolase A,
k) the tktB gene coding for transketolase B,
l) the gene product of the open reading frame (ORF) yddG of Escherichia coli,
m) the uspC gene coding for the universal stress protein UspC,
n) the uspD gene coding for the resistance against UV radiation-mediating universal stress protein UspD and
o) the uspE gene coding for the resistance against UV radiation-mediating universal stress protein UspE,
are overexpressed.

12. Process according to Claims 1 to 9, **characterized in that** for the production of L-threonine microorganisms are fermented, in which additionally at the same time one or more of the genes selected from the group consisting of:
a) the tdh gene coding for threonine dehydrogenase,
b) the mdh gene coding for malate dehydrogenase,
c) the pckA gene coding for phosphoenol pyruvate carboxykinase,
d) the poxB gene coding for pyruvate oxidase,
e) the dgsA gene coding for the DgsA regulator of the phosphotransferase system,
f) the fruR gene coding for the fructose repressor,
g) the rpoS gene coding for the Sigma³⁸ factor, and
h) the aspA gene coding for aspartate ammonium lyase,
are attenuated, in particular switched off, or the expression is decreased.

13. Process according to Claims 1 to 9 or 11, **characterized in that** for the production of L-tryptophan microorganisms of the family Enterobacteriaceae are fermented, in which additionally at the same time one or more of the genes selected from the group consisting of:
a) the tnaA gene coding for tryptophanase,
b) the trpR gene coding for the repressor of the trp operon,
c) the tyrA gene coding for chorismate mutase T and prephenate dehydrogenase,
d) the pheA gene coding for chorismate mutase P and prephenate dehydrogenase,
e) the mtr gene coding for the tryptophan-specific transport protein,
f) the tnaB gene coding for tryptophan permease,
g) the aroP gene coding for the transporter for aromatic amino acids,
h) the sdaA gene coding for L-serine deaminase,
i) the pgi gene coding for glucose-6-phosphate isomerase, and
j) the tyrB gene coding for tyrosine aminotransferase,
are attenuated, in particular switched off, or the expression is decreased.

14. Process according to Claims 1 to 9, **characterized in that** L-amino acids selected from the group consisting of L-threonine, L-tryptophan, L-lysine, L-valine, L-isoleucine and L-homoserine are produced.

15. Process according to Claims 1 to 14, **characterized in that** the microorganisms are cultured in a batch process, a fed batch process, a repeated fed batch process or in a continuous process.

16. Process according to Claims 1 to 15, **characterized in that** the concentration of the L-amino acid is determined at one or more point(s) in time in the course of the fermentation.

## Revendications

1. Procédé de préparation d'acides L-aminés par fermentation de micro-organismes recombinants de la famille des Enterobacteriaceae, qui contiennent un gène uspA surexprimé, lequel code pour la protéine de stress universelle A, **caractérisé en ce que**
a) on cultive les micro-organismes produisant l'acide L-aminé souhaité dans des conditions dans lesquelles l'acide L-aminé souhaité va subir un enrichissement dans le milieu ou dans les cellules, et
b) on isole l'acide L-aminé souhaité, des constituants du bouillon de fermentation et/ou la biomasse restant en totalité ou en partie (≥ 0 à 100 %) dans le produit isolé, ou étant entièrement éliminés, la surexpression du gène uspA étant réalisée dans les micro-organismes fermentés pour la préparation d'acides L-aminés par l'attachement de façon fonctionnelle au gène uspA d'un promoteur fort.

2. Procédé selon la revendication 1, **caractérisé en ce que**, pour préparer des acides L-aminés, on fermente des micro-organismes dans lesquels un polynucléotide correspondant au gène uspA est surexprimé, polynucléotide qui code pour un polypeptide dont la séquence d'acides aminés a une identité d'au moins 80 % avec une séquence d'acides aminés choisie dans le groupe SEQ ID N° 2, SEQ ID N° 4 et SEQ ID N° 6.

3. Procédé selon la revendication 1, **caractérisé en ce que**, pour la préparation d'acides L-aminés, on fermente des micro-organismes qui contiennent un polynucléotide isolé, correspondant au gène uspA, choisi dans le groupe consistant en :
a) un polynucléotide ayant une séquence nucléotidique choisie parmi SEQ ID N°1, SEQ ID N° 3, SEQ ID N° 5 et les séquences nucléotidiques qui lui sont complémentaires ;
b) un polynucléotide ayant une séquence nucléotidique qui, dans le cadre de la dégénérescence du code génétique, correspond à la séquence SEQ ID N° 1, SEQ ID N° 3 ou SEQ ID N° 5 ;
c) une séquence polynucléotidique ayant une séquence qui s'hybride dans des conditions stringentes à la séquence complémentaire de la séquence SEQ ID N° 1, SEQ ID N° 3 ou SEQ ID N° 5, les conditions stringentes étant atteintes grâce à une étape de lavage dans laquelle la température s'étend sur une plage de 64 à 68°C, et la concentration du sel dans le tampon s'étend sur une plage de 2xSSC à 0,1xSSC ;
d) un polynucléotide ayant une séquence SEQ ID N° 1, SEQ ID N° 3 ou SEQ ID N° 5, qui contient des mutants sens neutres d'un point de vue fonctionnel,
les polynucléotides codant pour la protéine de stress universelle A.

4. Procédé selon la revendication 2, **caractérisé en ce que**, pour la préparation d'acides L-aminés, on fermente des micro-organismes, **caractérisé en ce que** le polypeptide comprend une séquence d'acides aminés qui a une identité d'au moins 95 % avec l'une des séquences choisies dans le groupe SEQ ID N° 2, SEQ ID N° 4 et SEQ ID N° 6.

5. Procédé selon la revendication 2, **caractérisé en ce que**, pour la préparation d'acides L-aminés, on fermente des micro-organismes, **caractérisé en ce que** le polypeptide présente la séquence d'acides aminés qui a une identité de 100 % avec l'une des séquences SEQ ID N° 2 ou SEQ ID N° 4.

6. Procédé selon les revendications 1 à 5, **caractérisé en ce qu'**en tant que promoteur fort on utlise le promoteur tac, le promoteur trp, le promoteur lpp ou le promoteur P_{L} ou le promoteur P_{R} du phage λ ou les promoteurs du phage T7, les promoteurs gear-box, le promoteur nar ou les promoteurs des gènes rrsG rnpB, csrA, csrB, ompA, fusA, pepQ, rplX ou rpsG.

7. Procédé selon les revendications 1 à 6, **caractérisé en ce que**, pour la préparation d'acides L-aminés, on fermente des micro-organismes qui sont préparés par transformation, par transduction, par conjugaison ou par une combinaison de ces procédés, avec un vecteur qui contient le gène uspA, un allèle de ce gène, ou des parties de ce dernier et un promoteur.

8. Procédé selon les revendications 1 à 7, **caractérisé en ce que**, pour la préparation d'acides L-aminés, on fermente des micro-organismes, **caractérisé en ce que** les micro-organismes sont choisis parmi les genres Escherichia, Erwinia, Providencia et Serratia.

9. Procédé selon les revendications 1 à 8, **caractérisé en ce que**, pour la préparation d'acides L-aminés, on fermente des micro-organismes, **caractérisés en ce qu'**ils produisent de la L-thréonine, du L-tryptophane, de la L-lysine, de la L-valine, de la L-isoleucine, de la L-méthionine ou de la L-homosérine.

10. Procédé selon l'une des revendications 1 à 9, **caractérisé en ce que**, pour la préparation de L-thréonine, on fermente des micro-organismes dans lesquels on surexprime en outre, simultanément, un ou plusieurs des gènes choisis dans le groupe consistant en :
a) au moins un gène de l'opéron thrABC, codant pour l'aspartatekinase, l'homosérine-déshydrogénase, l'homosérinekinase et la thréoninesynthase,
b) la gène pyc de Corynebacterium glutamicum, codant pour la pyruvatecarboxylase,
c) le gène pps codant pour la phosphoénolpyruvate-synthase,
d) le gène ppc codant pour la phosphoénolpyruvate-carboxylase,
e) les gènes pntA et pntB codant pour les sous-unités de la pyrdine-transhydrogénase,
f) le gène rhtC, qui code pour la protéine conférant une résistance à la thréonine,
g) le gène thrE de Corynebacterium glutamicum, codant pour la protéine transporteuse d'exportation de la thréonine,
h) le gène gdhA codant pour la glutamate-déshydrogénase,
i) le gène ptsH codant pour la phosphohistidine-protéine-hexose-phosphotransférase,
j) le gène ptsI codant pour l'enzyme I du système phosphotransférase,
k) le gène crr codant pour le composant IIA spécifique du glucose,
l) le gène ptsG codant pour le composant IIBC spécifique du glucose,
m) le gène cysK codant pour la cystéinesynthase A,
n) le gène cysB codant pour le régulateur du régulon cys,
o) le gène cysJ codant pour la flavoprotéine de la NADPH-sulfite-réductase,
p) le gène cysI codant pour l'hémoprotéine de la NADPH-sulfite-réductase,
q) le gène cysH codant pour l'adénylylsulfate-réductase,
r) le gène sucA codant pour la sous-unité décarboxylase de la 2-cétoglutarate déshydrogénase,
s) le gène sucB codant pour la sous-unité dihydrolipoyltranssuccinase E2 de la 2-cétoglutarate déshydrogénase,
t) le gène sucC codant pour la sous-unité β de la succinyl-CoA synthétase,
u) le gène sucD codant pour la sous-unité α de la succinyl-CoA synthétase,
v) le produit génique du cadre de lecture ouvert (ORF) yjcG d'Escherichia coli,
w) le produit génique du cadre ouvert de lecture (ORF) yibD d'Escherichia coli,
x) le produit génique du cadre ouvert de lecture (ORF) yaaU d'Escherichia coli,
y) le gène uspC codant pour la protéine de stress universelle UspC,
z) le gène uspD, qui code pour la protéine de stress universelle UspD conférant une résistance au rayonnement UV, et
aa) le gène uspE, qui code pour la protéine de stress universelle UspE conférant une résistance au rayonnement UV.

11. Procédé selon l'une des revendications 1 à 9, **caractérisé en ce que**, pour la préparation de L-tryptophane, on fermente des micro-organismes de la famille des Enterobacteriaceae, dans lesquels on surexprime en plus et simultanément un ou plusieurs des gènes choisis dans le groupe comprenant :
a) au moins un gène de l'opéron tryptophane codant pour l'anthranilate-synthase, l'anthralinate-phosphoribosyl-transférase, la phosphoribosylanthranilate-isomérase, l'indole-3-glycérinephosphate-synthase, et la tryptophane-synthase,
b) le gène serA codant pour la 3-phosphoglycérate-déhydrogénase,
c) le gène serB codant pour la phosphosérine-phosphatase,
d) le gène serC codant pour la phosphosérine-aminotransférase,
e) le gène aroF codant pour la DHAP-synthase sensible à la L-tyrosine,
f) le gène aroG codant pour la DHAP-synthase sensible à la L-phénylalanine,
g) le gène aroH codant pour la DHAP-synthase sensible au L-tryptophane,
h) le gène pps codant pour la phosphoénolpyruvate-synthase,
i) le gène pckA codant pour la phosphoénolpyruvate-carboxykinase,
j) le gène tktA codant pour la transkétolase A,
k) le gène tktB codant pour la transkétolase B,
l) le produit génique du cadre ouvert de lecture (ORF) yddG d'Escherichia coli,
m) le gène uspC codant pour la protéine de stress universelle UspC,
n) le gène uspD codant pour la protéine de stress universelle UspD conférant une résistance au rayonnement UV, et
o) le gène uspE codant pour la protéine de stress universelle UspE conférant une résistance au rayonnement UV.

12. Procédé selon les revendications 1 à 9, **caractérisé en ce que**, pour la préparation de L-thréonine, on fermente des micro-organismes dans lesquels on affaiblit, en particulier on désactive, ou on en diminue l'expression, en plus et simultanément un ou plusieurs des gènes choisis dans le groupe consistant en :
a) le gène tdh codant pour la thréonine-déshydrogénase,
b) le gène mdh codant pour la malate-déshydrogénase,
c) le gène pckA codant pour la phosphoénolpyruvate-carboxykinase,
d) le gène poxB codant pour la pyruvate-oxydase,
e) le gène dgsA codant pour le régulateur DgsA du système phosphotransférase,
f) le gène fruR codant pour le répresseur fructose,
g) le gène rpoS codant pour le facteur Sigma³⁸, et
h) le gène aspA codant pour l'aspartate ammonium-lyase.

13. Procédé selon les revendications 1 à 9 ou 11, **caractérisé en ce que**, pour préparer le L-trytophane, on fermente des micro-organismes de la famille des Enterobacteriaceae, dans lesquels on affaiblit, en particulier on désactive, ou on en diminue l'expression, en plus et simultanément un ou plusieurs des gènes choisis dans le groupe consistant en :
a) le gène tnaA codant pour la tryptophanase,
b) le gène trpR codant pour le répresseur de l'opéron trp,
c) le gène tyrA codant pour la chorismate-mutase T et la préphénate-déshydrogénase,
d) le gène pheA codant pour la chorismate-mutase P et la préphénate-déshydrogénase,
e) le gène mtr codant pour la protéine transporteuse spécifique du tryptophane,
f) le gène tnaB codant pour la tryptophane-perméase,
g) le gène aroP codant pour le transporteur d'acides aminés aromatiques,
h) le gène sdaA codant pour la L-sérine désaminase,
i) le gène pgi codant pour la glucose-6-phosphate-isomérase, et
j) le gène tyrB codant pour la tyrosine-aminotransférase.

14. Procédé selon les revendications 1 à 9, **caractérisé en ce qu'**on prépare des acides L-aminés choisis dans le groupe consistant en la L-thréonine, le L-tryptophane, la L-lysine, la L-valine, la L-isoleucine et la L-homosérine.

15. Procédé selon les revendications 1 à 14, **caractérisé en ce que** les micro-organismes sont cultivés dans le cadre d'un procédé discontinu ou d'un procédé discontinu à alimentation programmée, d'un procédé discontinu à alimentation programmée répétée, ou d'un procédé continu.

16. Procédé selon les revendications 1 à 15, **caractérisé en ce qu'**on détermine la concentration de l'acide L-aminé à un ou plusieurs instants au cours de la fermentation.
